# EUROPEAN PATENT SPECIFICATION

(11) **EP 4 072 520 B1**
(45) Date of publication and mention of the grant of the patent: **24.09.2025**
(21) Application number: 20824345.1
(22) Date of filing: 07.12.2020
(51) Int. Cl.: A61K 9/00, A61K 9/20, A61K 47/26, A61K 47/34, A61P 31/18, A61K 39/00, A61K 31/4985, A61K 31/047, A61K 31/5365, A61K 31/517, A61K 31/52, A61K 31/519, A61K 31/541, A61K 31/77, C07K 16/10

(54) **PHARMACEUTICAL COMPOSITIONS COMPRISING CABOTEGRAVIR**
PHARMAZEUTISCHE ZUSAMMENSETZUNGEN MIT CABOTEGRAVIR
COMPOSITIONS PHARMACEUTIQUES CONTENANT DU CABOTÉGRAVIR

(30) Priority: 09.12.2019 US 201962945412 P; 27.02.2020 US 202062982305 P
(43) Date of publication of application: 19.10.2022
(73) Proprietor: VIIV Healthcare Company, Wilmington, DE 19808 (US)
(72) Inventor: AKHAVEIN, Nima, Collegeville, Pennsylvania 19426 (US); CHU, Kevin, Collegeville, Pennsylvania 19426 (US); VELTHUISEN, Emile, Collegeville, Pennsylvania 19426 (US)
(74) Representative: Telfer, Ruth Marie
(86) International application number: PCT/IB2020/061582
(87) International publication number: WO 2021/116872

(56) References cited:
- WO-A1-2015/127437
- WO-A1-2015/157483
- WO-A1-2016/046786
- WO-A1-2017/134596
- WO-A1-2018/002902
- WO-A1-2018/203235
- WO-A1-2019/171285
- WO-A1-2019/198024
- WO-A1-2019/207460
- WO-A1-2020/018459
- WO-A1-2020/112931
- WO-A1-2020/157692
- WO-A1-2020/222108
- WO-A1-2020/254985
- WO-A1-2021/050961
- WO-A2-2012/037320
- WO-A2-2017/223280
- US-A1- 2004 265 388
- US-A1- 2017 232 020
- US-A1- 2018 002 366
- ANONYMOUS: "ViiV Healthcare to develop N6LS for treatment and prevention of HIV By NS Healthcare Staff Writer22 Nov 2019", NS HEALTHCARE, 22 November 2019 (2019-11-22), XP055778457, Retrieved from the Internet <URL:https://www.ns-healthcare.com/news/viiv-develop-n6ls-hiv/> [retrieved on 20210222]
- ZHOU TIAN ET AL: "Creation of a nanoformulated cabotegravir prodrug with improved antiretroviral profiles", BIOMATERIALS, ELSEVIER, AMSTERDAM, NL, vol. 151, 15 October 2017 (2017-10-15), pages 53 - 65, XP085235415, ISSN: 0142-9612, DOI: 10.1016/J.BIOMATERIALS.2017.10.023
- TIAN ZHOU: "Next Generation of Translational Long-Acting Cabotegravir", THESES & DISSERTATIONS, 1 May 2018 (2018-05-01), University of Nebraska Medical Center Omaha, Nebraska, XP055708824, Retrieved from the Internet <URL:https://digitalcommons.unmc.edu/cgi/viewcontent.cgi?article=1261&context=etd> [retrieved on 20200625]
- "Cabotegravir Crystalline Form", RESEARCH DISCLOSURE, KENNETH MASON PUBLICATIONS, HAMPSHIRE, UK, GB, vol. 633, no. 64, 1 January 2017 (2017-01-01), pages 109, XP007145401, ISSN: 0374-4353, [retrieved on 20161219]
- MENDES E ET AL: "Synthesis, stability and in vitro dermal evaluation of aminocarbonyloxymethyl esters as prodrugs of carboxylic acid agents", BIOORGANIC & MEDICINAL CHEMISTRY, ELSEVIER, AMSTERDAM, NL, vol. 10, no. 3, 1 March 2002 (2002-03-01), pages 809 - 816, XP002296488, ISSN: 0968-0896, DOI: 10.1016/S0968-0896(01)00336-4
- MASAYUKI KAGEYAMA ET AL: "Enantioselective Total Synthesis of the Potent Anti-HIV Nucleoside EFdA", ORGANIC LETTERS ., vol. 13, no. 19, 7 October 2011 (2011-10-07), US, pages 5264 - 5266, XP055582155, ISSN: 1523-7060, DOI: 10.1021/ol202116k
- HARPER RACHEL: "Broadly neutralising antibody to be developed for HIV-1 treatment", EUROPEAN PHARMACEUTICAL REVIEW, 25 November 2019 (2019-11-25), pages 1 - 2, XP055795889, Retrieved from the Internet <URL:https://www.europeanpharmaceuticalreview.com/news/106488/investigational-broadly-neutralising-antibody-hiv-treatment/> [retrieved on 20210415]
- GROBBEN MARLOES ET AL: "The potential of engineered antibodies for HIV-1 therapy and cure", CURRENT OPINION IN VIROLOGY, vol. 38, 1 October 2019 (2019-10-01), United Kingdom, pages 70 - 80, XP055795898, ISSN: 1879-6257, DOI: 10.1016/j.coviro.2019.07.007
- ANONYMOUS: "Long-Acting Cabotegravir Plus VRC-HIVMAB075-00-AB (VRC07-523LS) for Viral Suppression in Adults Living With HIV-1", CLINICALTRIALS.GOV V IDENTIFIER: NCT03739996, 14 November 2018 (2018-11-14), pages 1 - 14, XP055795915, Retrieved from the Internet <URL:https://clinicaltrials.gov/ct2/show/NCT03739996> [retrieved on 20210415]
- JUCKER BEAT M ET AL: "Multimodal imaging approach to examine biodistribution kinetics of Cabotegravir (GSK1265744) long acting parenteral formulation in rat", JOURNAL OF CONTROLLED RELEASE, ELSEVIER, AMSTERDAM, NL, vol. 268, 16 October 2017 (2017-10-16), pages 102 - 112, XP085300178, ISSN: 0168-3659, DOI: 10.1016/J.JCONREL.2017.10.017
- "Handbook of Pharmaceutical Excipients", 1 January 2009, PHARMACEUTICAL PRESS, UK, ISBN: 978-0-85369-792-3, article ROWE R. C. ET AL: "Poloxamer", pages: 506 - 509, 651-6, XP055629883
- "Handbook of Pharmaceutical Excipients", 1 January 2006, PHARMACEUTICAL PRESS [U.A.], London [u.a.], ISBN: 978-1-58212-058-4, article RAYMOND C. ROWE ET AL: "Polyethylene Glycol", pages: 545 - 550, XP055399278, 031835

## Description

### FIELD OF THE INVENTION

The invention relates to Human Immunodeficiency Virus (HIV) treatment. In particular, the invention relates to long acting treatment of HIV.

### BACKGROUND TO THE INVENTION

Patients with HIV infection commonly undergo complex treatment regimens which involve taking multiple pills at regular intervals each day. Patient non-compliance is a known problem accompanying these complex HIV treatment regimens and can lead to the emergence of multiple-drug resistant strains of HIV.

The application of long-acting parenterals has been established in clinical practice for decades, notably in the areas of contraception, anti-psychotics and opiate addiction. More recently, long-acting parenterals have been explored for use in the treatment and prevention of HIV. Long-acting injectables have been proposed as a way of overcoming the non-compliance problem with HIV treatment regimens. Long-acting injectable formulations are in clinical development and clinical studies have demonstrated prolonged exposures (≥30 days) following injection, enabling dosing at once-monthly intervals.

Achieving an injectable suspension with a high concentration of anti-HIV drug in order to dose less frequently and overcome the non-compliance problem with HIV treatment regimens, whilst keeping the same injection volume as previous HIV treatment regimens in order to maintain patient experience is desirable. High concentration suspensions typically suffer from difficulty in resuspension and particle size growth. WO 2017/223280 A2 discloses integrase inhibitor prodrugs, including nanoparticles comprising at least one integrase inhibitor prodrug and at least one surfactant. WO 2016/046786 A1 discloses long acting pharmaceutical compositions or pharmaceutically acceptable salts thereof, useful in the treatment or prevention of Human Immunodeficiency Virus (HIV) infections. WO 2015/127437 A1 discloses nanoparticles/nanoformulations comprising at least one therapeutic agent and at least one surfactant. WO 2012/037320 A2 discloses pharmaceutical compositions of cabotegravir useful in the treatment or prevention of HIV infections.

There is a need in the art for a long acting injectable to treat HIV that can be dosed at fewer intervals whilst still achieving the same patient experience and overcoming know difficulties with high concentration suspensions.

### SUMMARY OF THE INVENTION

Aspects of the invention are defined in claims 1 to 16.

Any reference to methods of treatment by therapy of this description is to be interpreted as a reference to compounds, pharmaceutical compositions and medicaments of the present invention for use in such methods.

The compositions of the invention may be advantageous in a number of respects. The compositions of the invention allow a high concentration of cabotegravir to be present in the composition. Currently there are no marketed suspensions with drug concentration higher than 312 mg/mL. When considering marketed suspensions for any pharmaceutical use, the suspensions with the highest drug concentrations are ARISTADA INITIO (aripipazole lauroxil, used to treat adults with schizophrenia) and INVEGA TRINZA (paliperidone palmitate, used to treat adults with schizophrenia) which have solid drug concentrations of 281.25 mg/mL and 312 mg/mL respectively. The compositions of the present invention enable up to 600 mg/mL of cabotegravir to be present in the composition (for example about 400 mg/mL).

The high concentration of cabotegravir allowed by the compositions of the present invention allows the concentrations to be administered to a patient less frequently than known HIV treatments. Compositions of the present invention also exhibit particle size stability and are able to achieve resuspension after storage.

### DESCRIPTION OF DRAWINGS/FIGURES

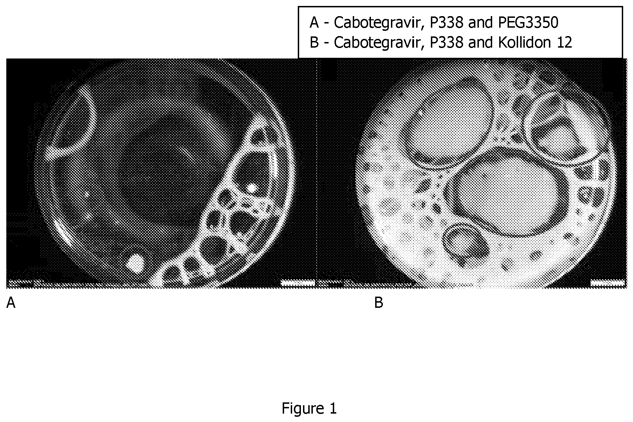

Figure 1 shows a comparison of resuspension of two suspensions, Figure 1 A shows a suspension comprising cabotegravir, P338 (poloxamer) and PEG3350 (left) and Figure 1 B shows a suspension comprising cabotegravir, P338 and Kollidon 12 (polyvinylpyrrolidone) (right). The left vial shows a mostly clear vial bottom indicating that the suspension was resuspended and decanted. The right vial shows a vial bottom with suspension adhered to the bottom of the vial, indicating that resuspension was not achieved.

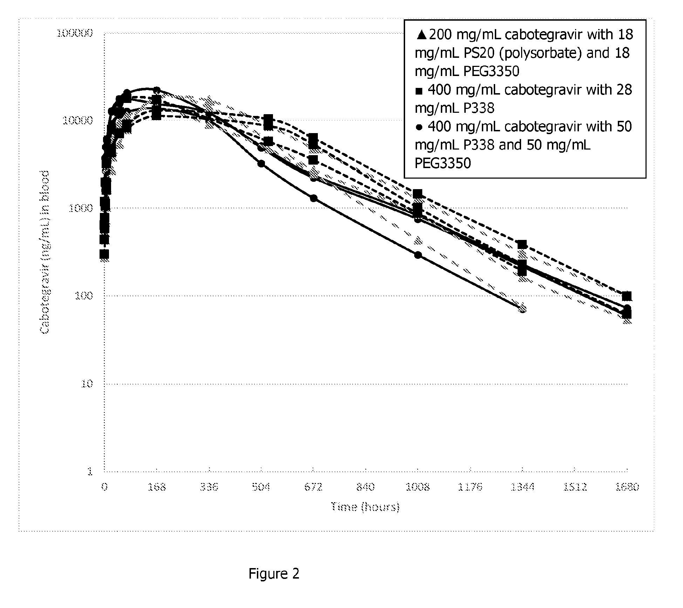

Figure 2 shows cabotegravir concentrations in blood of individual rats after a 10 mg/kg intramuscular administration of: a 200 mg/mL cabotegravir with 18 mg/mL PS20 (polysorbate) and 18 mg/mL PEG3350 (▲); 400 mg/mL cabotegravir with 28 mg/mL P338 (▪); or 400 mg/mL cabotegravir with 50 mg/mL P338 and 50 mg/mL PEG3350 (•). Formulations also contained mannitol (14 to 30 mg/mL) for tonicity adjustment. 3 rats were used per formulation.

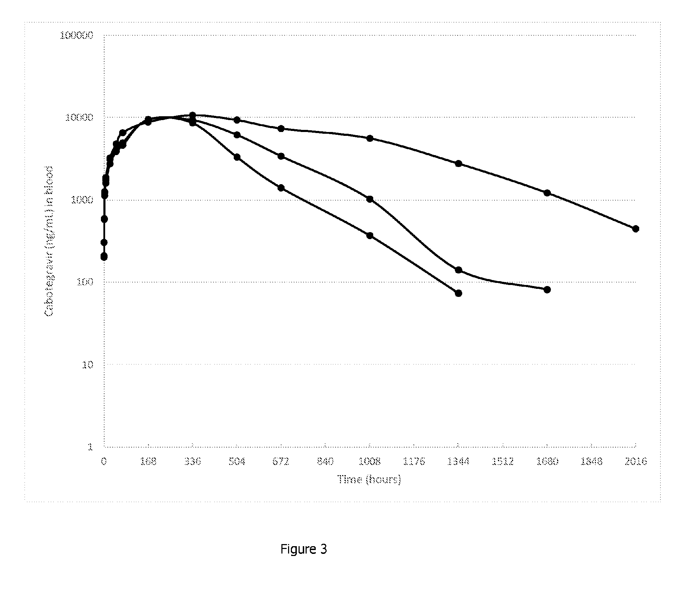

Figure 3 shows cabotegravir concentrations in blood of individual rats after a 10 mg/kg subcutaneous administration of 400 mg/mL cabotegravir with 50 mg/mL P338, 50 mg/mL PEG3350 and 30 mg/mL mannitol (•). 3 rats were used.

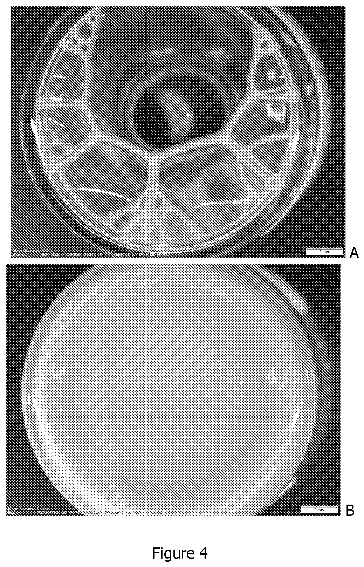

Figure 4 shows microscopy images of suspensions. Figure 4A (left) shows suspension 3a which was fully resuspended after storage for 6 months upright at 30°C/65%RH. Figure 4B (right) shows suspension 2a which was not resuspended after storage for 6 months upright at 30°C/65%RH.

### DETAILED DESCRIPTION OF THE INVENTION

### DEFINITIONS

As used herein, the term 'pharmaceutical composition' means a composition that is suitable for pharmaceutical use.

As used herein, the term "pharmaceutically acceptable salts" refers to salts that retain the desired biological activity of the subject compound and exhibit minimal undesired toxicological effects. These pharmaceutically acceptable salts may be prepared *in situ* during the final isolation and purification of the compound, or by separately reacting the purified compound in its free acid or free base form with a suitable base or acid, respectively.

Pharmaceutically acceptable salts include, amongst others, those described in Berge, J. Pharm. Sci., 1977, 66, 1-19, or those listed in P H Stahl and C G Wermuth, editors, Handbook of Pharmaceutical Salts; Properties, Selection and Use, Second Edition Stahl/Wermuth: Wiley-VCH/VHCA, 2011 (see http://www.wiley.com/WileyCDA/WileyTitle/productCd-3906390519.html). Suitable pharmaceutically acceptable salts can include acid or base addition salts. Suitable pharmaceutically acceptable salts of the invention include base addition salts.

Representative pharmaceutically acceptable base addition salts include, but are not limited to, aluminium, 2-amino-2-(hydroxymethyl)-1,3-propanediol (TRIS, tromethamine), arginine, benethamine (*N-*benzylphenethylamine), benzathine (*N,N'-*dibenzylethylenediamine), *bis-(2-*hydroxyethyl)amine, bismuth, calcium, chloroprocaine, choline, clemizole (1-p chlorobenzyl-2-pyrrolildine-1'-ylmethylbenzimidazole), cyclohexylamine, dibenzylethylenediamine, diethylamine, diethyltriamine, dimethylamine, dimethylethanolamine, dopamine, ethanolamine, ethylenediamine, L-histidine, iron, isoquinoline, lepidine, lithium, lysine, magnesium, meglumine (*N-*methylglucamine), piperazine, piperidine, potassium, procaine, quinine, quinoline, sodium, strontium, t-butylamine, and zinc.

As used herein, the term "treatment" or "treating" refers to alleviating the specified condition, eliminating or reducing the symptoms of the condition, slowing or eliminating the progression, invasion, or spread of the condition and reducing or delaying the reoccurrence of the condition in a previously afflicted subject.

As used herein, the term "prevention" or "preventing" refers to precluding the specified condition or symptoms of the condition.

### STATEMENT OF THE INVENTION

Disclosed herein is a pharmaceutical composition comprising cabotegravir or a pharmaceutically acceptable salt thereof, polyethylene glycol and poloxamer.

Disclosed herein, the pharmaceutical compositions described herein may be administered by any appropriate route. In a preferred embodiment, the compositions are administered parenterally (including subcutaneous, intramuscular, intravenous, or intradermal). In one embodiment the composition is administered intramuscularly. In another embodiment the composition is administered subcutaneously.

Cabotegravir (*N-*((2,4-Difluorophenyl)methyl)-6-hydroxy-3-methyl-5,7-dioxo-2,3,5,7,11,11a hexahydro(1,3)oxazolo(3,2-a)pyrido(1,2-d)pyrazine-8-carboxamide) is described in US 8,129,385 in example Z-1, which example is incorporated herein by reference. Cabotegravir is an integrase strand transfer inhibitor (INSTI) that exhibits subnanomolar potency and antiviral activity against a broad range of HIV-1 strains. Oral administration of Cabotegravir has exhibited acceptable safety and tolerability profiles, a long half-life, and few drug-drug interactions. Cabotegravir has been demonstrated to be efficacious in treatment and prevention of HIV both in oral and parenteral dosage forms, see for instance, Margolis DA, Brinson CC, Eron JJ, et al. 744 and Rilpivirine as Two Drug Oral Maintenance Therapy: LAI116482 (LATTE) Week 48 Results. 21st Conference on Retroviruses and Opportunistic Infections (CROI); March 3-6, 2014; Boston, MA, Margolis DA, Podzamczer D, Stellbrink H-J, et al. Cabotegravir + Rilpivirine as Long-Acting Maintenance Therapy: LATTE-2 Week 48 Results. 21st International AIDS Conference; July 18-22, 2016; Durban, South Africa, Abstract THAB0206LB. Levin: Conference reports for National AIDS Treatment Advocacy Project (NATAP); 2016, and Markowitz M, Frank I, Grant R, et al. ECLAIR: Phase 2A Safety and PK Study of Cabotegravir LA in HIV-Uninfected Men. Abstract presented at: 23rd Conference on Retroviruses and Opportunistic Infections (CROI); February 22-25, 2016; Boston, MA.

Cabotegravir is represented by formula (I):

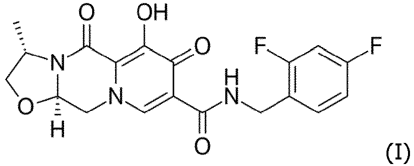

In an embodiment of the invention, cabotegravir is present in the pharmaceutical composition as the free acid.

The pharmaceutical compositions disclosed herein comprise a poloxamer. Poloxamers are nonionic triblock copolymers composed of a central hydrophobic chain of polyoxypropylene (polypropylene oxide) flanked by two hydrophilic chains of polyoxyethylene (polyethylene glycol). Poloxamers are represented by Formula II. Poloxamers are also known by the trade names Synperonics, Pluronics, and Kolliphor. Poloxamers are commonly named with the letter "P" followed by three digits, the first two digits x 100 give the approximate molecular mass of the polyoxypropylene core, and the last digit x 10 gives the percentage polyoxyethylene content (e.g., P407 = Poloxamer with a polyoxypropylene molecular mass of 4,000 g/mol and a 70% polyoxyethylene content).

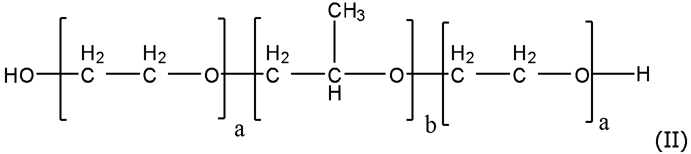

Disclosed herein, the poloxamer may have a molecular mass of polyoxypropylene from 2000 to 5000 g/mol, and a polyoxyethylene content of 60% to 90%. Disclosed herein, the poloxamer may be P237, P338 or P407 (a and b values for these poloxamers can be seen in Table 1). P237, P338 and P407 are commercially available.

**Table 1**

| **Poloxamer** | **a** | **b** |
|---|---|---|
| P237 | 64 | 37 |
| P338 | 141 | 44 |
| P407 | 101 | 56 |

Disclosed herein, the poloxamer is P237. Disclosed herein, the poloxamer is P407. In embodiments the poloxamer is P338.

It is desirable to have a high concentration of cabotegravir in the composition (for example a cabotegravir concentration of around 400 or around 500 mg/mL). The inventors have found that the use of poloxamer in the composition allows a high concentration of cabotegravir to be present in the pharmaceutical composition. Cabotegravir is present in the composition in particle form. The inventors have found that at high particle concentrations, there are more particle to particle interactions. These particle to particle interactions can lead to an unstable suspension. Poloxamer has both hydrophobic and hydrophilic chains. It is thought that the hydrophobic polyoxypropylene chains of poloxamer adsorb onto the surface of the cabotegravir particles and thus provide steric hinderance between particles. This provides stabilization against particle aggregation or flocculation. It is thought that the polyethylene glycol chains provide stabilisation through steric hinderance, thus allowing a higher concentration of cabotegravir to be present in the overall composition and minimising the particle to particle interactions usually associated with high particle concentrations.

The inventors have found that the use of poloxamer P237, P407 or P338 gives enhanced physical stability to the pharmaceutical compositions of the invention. Without wishing to be bound by theory, it is thought this enhanced stability comes from the fact that P237, P407 and P338 comprise more polyethylene glycol chains than other commercially available poloxamers.

The pharmaceutical compositions disclosed herein also comprise polyethylene glycol (PEG). PEG is a polymer of ethylene oxide and is represented by formula (III): 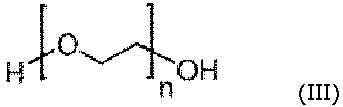 Disclosed herein, n may be any suitable number. Disclosed herein, PEG has a number average mean molecular weight (Mₙ) of from 1000 to 8000 g/mol. Disclosed herein, PEG has an Mₙ of from 2500 to 5000 g/mol. Disclosed herein, PEG has an Mₙ of from 3000 to 4000 g/mol. Disclosed herein, PEG has an Mₙ of 3100 to 3700 g/mol. In embodiments of the invention, PEG is PEG 3350 *i.e.* PEG has an Mₙ of 3350 g/mol. PEG3350 is commercially available.

Without wishing to be bound by theory, the inventors believe that PEG acts as a stabilizer in the compositions of the present invention, further stabilizing the cabotegravir alongside the PEG chains that are present in the poloxamer. As a non-adsorbing polymer, it is thought that PEG stabilizes the compositions of the present invention through increase in viscosity and providing steric repulsions between particles in the suspension.

The combination of PEG and poloxamer enables the high cabotegravir concentration described above to be achieved. This enables the composition of the invention to treat HIV in a patient for up to 6 months. In an embodiment the composition of the invention can treat HIV in a patient for up to 3 months, therefore enabling dosing once every month, 2 months or 3 months. In an embodiment about 1 mL to 3 mL of the pharmaceutical composition is delivered to a patient. In comparison with known and proposed cabotegravir compositions, the present invention allows for a higher dose to be delivered to the patient with less frequent dosing while keeping injection volume the same as for previous products.

Disclosed herein, the composition comprises cabotegravir, polyethylene glycol 3350 and poloxamer 407. In embodiments of the invention, the pharmaceutical composition comprises cabotegravir, polyethylene glycol 3350 and poloxamer 338. Also disclosed herein, the pharmaceutical composition comprises cabotegravir, polyethylene glycol 3350 and poloxamer 237.

In embodiments of the invention, the pharmaceutical composition comprises from 350 to 600 mg/mL of cabotegravir. In an embodiment of the invention, the pharmaceutical composition comprises from 350 to 500 mg/mL of cabotegravir. In another embodiment, the pharmaceutical composition comprises 380 to 420 mg/mL of cabotegravir. In another embodiment, the pharmaceutical composition comprises about 400 mg/mL of cabotegravir. In an alternative embodiment the pharmaceutical composition comprises about 500 mg/mL of cabotegravir

In an embodiment, the pharmaceutical composition comprises a poloxamer concentration of from 4 to 50 mg/mL. In another embodiment the pharmaceutical composition comprises a poloxamer concentration of from 20 to 50 mg/mL. In an embodiment, the pharmaceutical composition comprises a poloxamer concentration of from 30 to 40 mg/mL. In another embodiment, the pharmaceutical composition comprises a poloxamer concentration of from 35 to 40 mg/mL.

As described above, the PEG chains in poloxamer provide stabilisation by steric hinderance, therefore it is possible that 0 mg/mL of PEG may be present in a composition disclosed herein. However, a composition comprising poloxamer and PEG provides additional stability to the composition, particularly particle size stability, and is preferred. A pharmaceutical composition comprising a polyethylene glycol concentration of 0 to 50 mg/mL is disclosed herein.

Disclosed herein, the pharmaceutical composition comprises a polyethylene glycol concentration of from more than 0 to 75 mg/mL. In embodiments, the pharmaceutical composition comprises a PEG concentration of 5 to 50 mg/mL. In another embodiment, the pharmaceutical composition comprises a polyethylene glycol concentration of from 20 to 40 mg/mL. In another embodiment, the pharmaceutical composition comprises a polyethylene glycol concentration of from 30 to 40 mg/mL. In another embodiment, the pharmaceutical composition comprises a polyethylene glycol concentration of from 32 to 38 mg/mL.
Disclosed herein, the pharmaceutical composition comprises cabotegravir or a pharmaceutically acceptable salt thereof, >0 to 75 mg/mL of PEG and 4 to 50 mg/mL of poloxamer. Disclosed herein is the pharmaceutical composition as described in Table 2a:

**Table 2a**

| **Ingredient** | **Concentration (mg/mL)** |
|---|---|
| Cabotegravir | About 400.0 |
| Poloxamer 338 | 4 - 50 |
| Polyethylene Glycol 3350 | >0 - 75 |
| Mannitol | 20 (Adjusted to be isotonic within 285 - 310 mOsmol/kg) |

In an embodiment, the pharmaceutical composition is as described in Table 2b:

**Table 2b**

| **Ingredient** | **Concentration (mg/mL)** | **Lower allowed Concentration (mg/mL)** | **Higher allowed Concentration (mg/mL)** |
|---|---|---|---|
| Cabotegravir | 400 | 320 | 500 |
| Poloxamer 338 | 38 | 30 | 47.5 |
| Polyethylene Glycol 3350 | 35 | 28 | 43.7 |
| Mannitol | 20 | 16 | 25 |

Further disclosed is the composition as described in Table 2c:

**Table 2c**

| **Ingredient** | **Concentration (mg/mL)** | |
|---|---|---|
| | | |
| Cabotegravir | About 500.0 | About 500.0 |
| Poloxamer 338 | 35 - 50 | -- |
| Poloxamer 407 | -- | 35 - 50 |
| Polyethylene Glycol 3350 | >0 - 75 | >0 - 75 |
| Mannitol | 40 (Adjusted to be isotonic within 285 - 310 mOsmol/kg) | 40 (Adjusted to be isotonic within 285 - 310 mOsmol/kg) |

The pharmaceutical composition of the present invention comprises particles of crystalline cabotegravir. In an embodiment the median particle diameter is from 0.1 µm to 10.0 µm. In another embodiment the median particle diameter is between 0.1 µm and 0.4 µm. In another embodiment the median particle diameter is from 0.15 µm to 0.25 µm. In another embodiment the median particle diameter is about 0.2 µm. The inventors have found that compositions with a median particle diameter of from 0.15 µm to 0.25 µm *e.g.* of about 0.2 µm have improved resuspension over compositions with larger particle sizes (as used here 'resuspension' means dispersion of sedimented product to a free-flowing suspension). The median particle diameter may be measured by any suitable method, for example, median particle diameter may be measured by laser diffraction (Malvern Mastersizer 3000) as described in the Examples section herein.

Pharmaceutical compositions of the present invention may retain their particle size over time. This is advantageous as increase in particle size can lead to poor resuspension. Poor resuspension can, for example, make it difficult for healthcare professionals to withdraw a pharmaceutical composition from, for example, a vial or flask. Particle size stability is believed to be important for resuspension of the composition over time and reduces the risk of changing pharmacokinetics.

Disclosed herein, the pharmaceutical composition comprises cabotegravir or a pharmaceutically acceptable salt thereof, >0 to 75 mg/mL of PEG and 4 to 50 mg/mL of poloxamer, wherein the median particle diameter of cabotegravir is from 0.1 µm to 10.0 µm.

Disclosed herein, the pharmaceutical composition comprises cabotegravir or a pharmaceutically acceptable salt thereof, >0 to 75 mg/mL of PEG and 4 to 50 mg/mL of poloxamer, wherein the median particle diameter of cabotegravir is from 0.15 µm to 0.25 µm.

In an embodiment the composition of the invention further comprises a tonicity adjuster. The tonicity adjuster may be selected from any suitable tonicity adjuster. In an embodiment of the invention, the tonicity adjuster adjusts tonicity to be within 250 to 350 mOsmol/kg. In another embodiment the tonicity adjuster adjusts tonicity to be within 285 to 310 mOsmol/kg. In an embodiment mannitol is used as the tonicity adjuster. In an embodiment of the invention, the mannitol adjusts tonicity to be within 250 to 350 mOsmol/kg. In another embodiment the mannitol adjusts tonicity to be within 285 to 310 mOsmol/kg. In an embodiment of the invention mannitol is present in the pharmaceutical composition at a concentration of 15 to 30 mg/mL.

In an embodiment the composition of the invention has a pH of about 4 or greater. A pH of about 4 or more reduces pain to the patient if the composition is administered via injection.

In an embodiment the pharmaceutical composition of the present invention further comprises at least one hyaluronidase. As used herein, the term 'hyaluronidase' refers to any enzyme of the family of hyaluronidases that catalyse the degradation of hyaluronic acid, a constituent of the extracellular matrix. Degradation of hyaluronic acid causes subcutaneous and intramuscular tissues to become more permeable allowing fluid or medication to diffuse into the extracellular matrix and be taken up by the surrounding tissue more quickly. Without wishing to be bound by theory, it is thought that administrating a pharmaceutical composition of the present invention in combination with a hyaluronidase may allow administration of a larger volume of the pharmaceutical composition thus allowing dosing frequency to be reduced. In addition, it is believed that the use of at least one hyaluronidase will provide an improved tolerability for the pharmaceutical composition at the site of administration.

The disclosure provides a method for the treatment or prevention of human immunodeficiency virus (HIV) in a human in need thereof comprising administering to said human a therapeutically effective amount of a pharmaceutical composition as defined herein.

In one embodiment the method comprises administering the pharmaceutical composition parenterally. In an embodiment the pharmaceutical composition is administered intramuscularly. In an embodiment the pharmaceutical composition is administered subcutaneously.

In an embodiment around 1mL to around 3 mL of the pharmaceutical composition is administered to the human. In an embodiment around 1mL of the pharmaceutical composition is administered to the human. In another embodiment around 2 mL of the pharmaceutical composition is administered to the human. In an embodiment around 3 mL of the pharmaceutical composition is administered to the human.

In an embodiment the pharmaceutical composition is administered to the human once every 1, 2 or 3 months. In an embodiment the pharmaceutical composition is administered to the human once every month. In an alternative embodiment, the pharmaceutical composition is administered once every 2 months. In an alternative embodiment the pharmaceutical composition is administered once every 3 months.

In one embodiment, the pharmaceutical composition is delivered to a patient once every month. Disclosed herein, the pharmaceutical composition may be administered by any suitable means. In one embodiment, the pharmaceutical composition is self-administered by a patient. The term "self-administered", as used herein, means administration by someone other than a healthcare professional, for example, a patient may administer the pharmaceutical composition to themselves, or someone else, other than a healthcare professional may administer the pharmaceutical composition to the patient. In this embodiment the pharmaceutical composition may be administered subcutaneously via injection. The subcutaneous injection may comprise 1 to 1.5 mL of the pharmaceutical composition. In one embodiment of the invention the pharmaceutical composition is self-administered once monthly by subcutaneous injection.

In an alternative embodiment the pharmaceutical composition is delivered to a patient once every three months. Disclosed herein, the pharmaceutical composition may be administered by any suitable means. In one embodiment the pharmaceutical composition is administered intramuscularly via injection. In an embodiment the intramuscular injection is administered by a healthcare professional. The intramuscular injection may comprise 1 to 3 mL of the pharmaceutical composition. In an embodiment of the invention the pharmaceutical composition is administered once every 3 months via intramuscular injection.

The pharmaceutical compositions of the present invention may be administered in combination with other pharmaceutical compositions as a component of a multi drug treatment regimen. The other pharmaceutical compositions may be drugs which treat or prevent HIV. Marketed medicines are currently available to treat HIV.

In an embodiment a pharmaceutical compound described herein is administered in combination with a broadly neutralising antibody. In an embodiment the neutralising antibody is N6-LS. N6-LS is a broadly neutralising antibody, which comprises (a) a heavy chain variable region (VH) comprising a heavy chain complementarity determining region (HCDR)1, a HCDR2, and a HCDR3 of the VH set forth as SEQ ID NO: 1; (b) a light chain variable region (VL) comprising a light chain complementarity determining region (LCDR) 1, a LCDR2, and a LCDR3 of the VL set forth as SEQ ID NO: 2 or a VL comprising an amino acid sequence at least 90 percent identical to one of SEQ ID NO: 2; or (c) a combination of (a) and (b); further comprising an IgGI constant domain comprising M428L and N434S mutations; and wherein the antibody or antigen binding fragment specifically binds to HIV-1 gpl20 and neutralizes HIV-1 infection.

SEQ ID NO: 1 is the amino acid sequence of the VH of the N6 mAb disclosed in patent application PCT/US2016/023145. SEQ ID NO: 2 is the amino acid sequence of the VL of the N6 mAb disclosed in patent application PCT/US2016/023145. SEQ ID NO: 1 and SEQ ID NO: 2 are described below:

SEQ ID NO 1:



SEQ ID NO: 2:



In an embodiment, a pharmaceutical composition described herein is administered in combination with a capsid inhibitor, a maturation inhibitor or a nucleoside reverse transcriptase translocation inhibitor (NRTTI).

The pharmaceutical composition may be administered in combination with a capsid inhibitor. In an embodiment the capsid inhibitor is a compound of Formula I, or a pharmaceutically acceptable salt thereof: 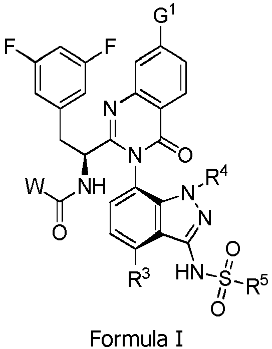 wherein:
G1 is phenyl substituted once with -N(CH3)S(O2)CH3, -S(O2)C(CH3)3, -CHF2, -CF3, -OCHF2, - OCF3, or -C(CH3)2OH, with the proviso that when G1 is -CHF2 or CF3, G1 is not in the para position or G1 is one of the following: 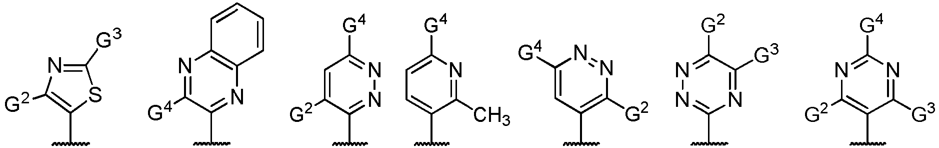 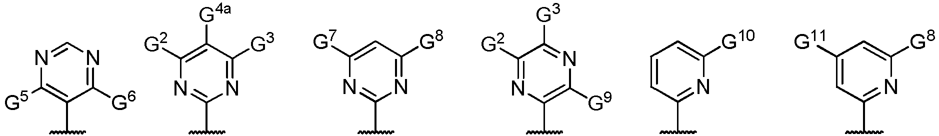 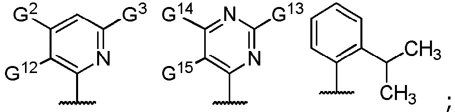
G² and G³ are independently selected from is H or -CH₃;
G⁴ is H, -CH₃, or -OCH₃;
G^{4a} is -CH₃, **or** -OCH₃;
G⁵ is -CH₃, or CH₂CH₃;
G⁶ is H, -CH₃, or CH₂CH₃;
G⁷ is ethyl, isopropyl, tert-butyl, -CHF₂, or -CF₃;
G⁸ is H, methyl, ethyl, -CHF₂, -CF₃, -OCH₃, or -OCH₂CH₃;
G⁹ is ethyl, isopropyl, cyclopropyl, -CH₂OH, -OCH₃;
G¹⁰ is ethyl, isopropyl, cyclopropyl, tert-butyl, -CHF₂, or -CF₃;
G¹¹ is methyl, -OCH₃, -CHF₂, -CF₃, -S(O₂)CH₃;
G¹² is F, -CH₃, -CHF₂, -CF₃, -OCH₃, -S(O₂)CH₃;
G¹³ is C₁-C₄alkyl, C₁-C₆cycloalkyl, -CH₂O(C₁-C₃alkyl);
G¹⁴ is H, C₁-C₄alkyl, -CHF₂, -CF₃, -O(C₁-C₃alkyl);
G¹⁵ is H, F, -CH₃, or OCH₃;
R³ is H, F, Cl, -CH₃, or -OCH₃;
R⁴ is H or C₁-C₃alkyl wherein C₁-C₃alkyl is optionally substituted with 1-3 fluorines;
R⁵ is C₁-C₆ alkyl or C₃-C₆ cycloalkyl;
W is selected from: 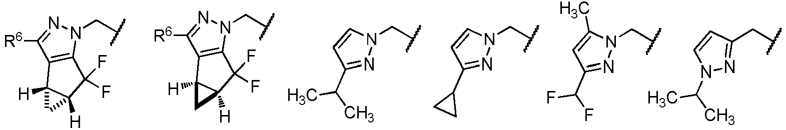 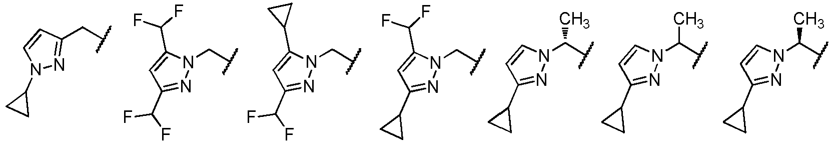 where R⁶ is methyl optionally substituted with 1 to 3 fluorines.

Compounds of Formula I are described in WO2020/084492 which is incorporated by reference. In an embodiment the capsid inhibitor is Compound 1 or a pharmaceutically acceptable salt thereof. Compound 1 is described in WO 2020/084492 as Example 59 which example is incorporated herein by reference.

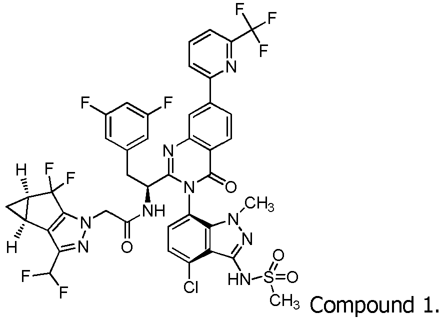

In an embodiment the capsid inhibitor is Compound 2 pharmaceutically acceptable salt thereof. Compound 2 is described in patent application number PCT/IB2020/055653 as Example 1, which example is incorporated herein by reference.

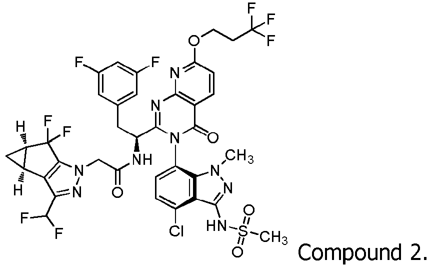

In an alternative embodiment, the capsid inhibitor is Lenacapravir.

The pharmaceutical composition may be administered in combination with a maturation inhibitor. In an embodiment the maturation inhibitor is a compound of Formula II or a pharmaceutically acceptable salt thereof: 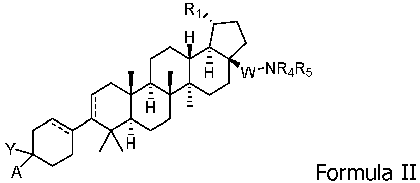
wherein R₁ is isopropenyl or isopropyl;
A is -C₁₋₆ alkyl-OR₀;
wherein R₀ is heteroaryl-Q₀;
Q₀ is selected from the group of -H, -CN, -C₁₋₆ alkyl, -COOH, -Ph, -OC₁₋₆ alkyl, -halo, -CF₃,
Y is selected from the group of -COOR₂, -C(O)NR₂SO₂R₃, -C(O)NHSO₂NR₂R₂, - SO₂NR₂C(O)R₂, -tetrazole, and -CONHOH,
wherein n = 1-6;
R₂ is -H, -C₁₋₆ alkyl, -alkylsubstituted C₁₋₆ alkyl or-arylsubstituted C₁₋₆ alkyl;
W is absent, or is -CH₂- or -CO-;
R₃ is -H, -C₁₋₆ alkyl or -alkylsubstituted C₁₋₆ alkyl;
R₄ is selected from the group of -H, -C₁₋₆ alkyl, -C₁₋₆ alkyl-C₃₋₆ cycloalkyl, -C₁₋₆ substituted -C₁₋₆ alkyl, -C₁₋₆ alkyl-Q₁, -C₁₋₆ alkyl-C₃₋₆ cycloalkyl-Q₁, aryl, heteroaryl, substituted heteroaryl, -COR₆, - SO₂R₇, -SO₂NR₂R₂, and 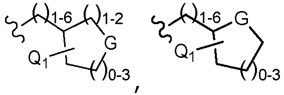
wherein G is selected from the group of -0-, -SO₂- and -NR₁₂-;
wherein Q₁ is selected from the group of -C₁₋₆ alkyl, - C₁₋₆ fluoroalkyl, heteroaryl, substituted heteroaryl, halogen, -CF₃, -OR₂, -COOR₂, -NR₈R₉, -CONR₈R₉ and -SO₂R₇;
R₅ is selected from the group of -H, -C₁₋₆ alkyl, -C₃₋₆ cycloalkyl, -C₁₋₆ alkylsubstituted alkyl, -C₁₋₆ alkyl-NR₈R₉, -COR₃, -SO₂R₇ and -SO₂NR₂R₂;
with the proviso that R₄ or R₅ is not -COR₆ when W is -CO-;
with the further proviso that only one of R₄ or R₅ is selected from the group of -COR₆, -COCOR₆,-SO₂R₇ and -SO₂NR₂R₂;
R₆ is selected from the group of -H, -C₁₋₆ alkyl, -C₁₋₆ alkyl-substitutedalkyl, -C₃₋₆ cycloalkyl, -C₃₋₆ substitutedcycloalkyl-Q₂, -C₁₋₆ alkyl-Q₂, -C₁₋₆ alkyl-substitutedalkyl-Q₂,-C₃₋₆ cycloalkyl-Q₂, aryl-Q₂, - NR₁₃R₁₄, and -OR₁₅;
wherein Q₂ is selected from the group of aryl, heteroaryl, substituted heteroaryl, -OR₂, -COOR₂, - NR₈R₉, SO₂R₇, -CONHSO₂R₃, and -CONHSO₂NR₂R₂;
R₇ is selected from the group of -H, -C₁₋₆ alkyl, -C₁₋₆ substituted alkyl, -C₃₋₆ cycloalkyl, -CF₃, aryl, and heteroaryl;
R₈ and R₉ are independently selected from the group of -H, -C₁₋₆ alkyl, -C₁₋₆ substituted alkyl, aryl, heteroaryl, substituted aryl, substituted heteroaryl, -C₁₋₆ alkyl-Q₂, and -COOR₃,
or R₈ and R₉ are taken together with the adjacent N to form a cycle selected from the group of: 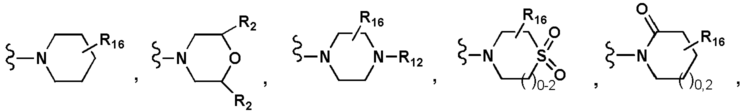 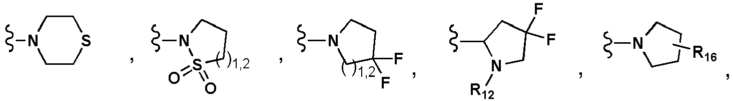 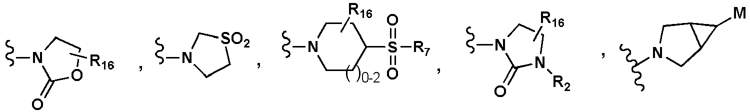 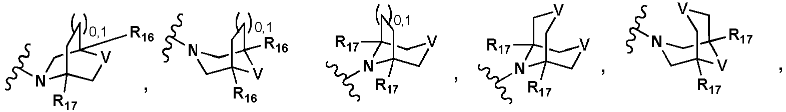 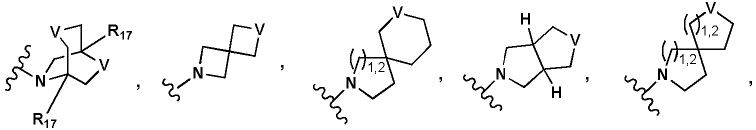 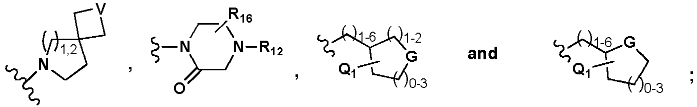
M is selected from the group of -R₁₅, -SO₂R₂, -SO₂NR₂R₂, -OH and -NR₂R₁₂;
V is selected from the group of -CR₁₀R₁₁-, -SO₂-, -O- and -NR₁₂-;
with the proviso that only one of R₈ or R₉ can be -COOR₃;
R₁₀ and R₁₁ are independently selected from the group of -H, -C₁₋₆ alkyl, -C₁₋₆ substituted alkyl and -C₃₋₆ cycloalkyl;
R₁₂ is selected from the group of -H, -C₁₋₆ alkyl, -alkylsubstituted C₁₋₆ alkyl, -CONR₂R₂, -SO₂R₃, and -SO₂NR₂R₂;
R₁₃ and R₁₄ are independently selected from the group of -H, -C₁₋₆ alkyl, -C₃₋₆ cycloalkyl, -C₁₋₆ substituted alkyl, -C₁₋₆ alkyl-Q₃, -C₁₋₆ alkyl-C₃₋₆ cycloalkyl-Q₃, and C₁₋₆ substituted alkyl-Q₃;
Q₃ is selected from the group of heteroaryl, substituted heteroaryl, -NR₂R₁₂, -CONR₂R₂, -COOR₂, - OR₂, and -SO₂R₃;
R₁₅ is selected from the group of -C₁₋₆ alkyl, -C₃₋₆ cycloalkyl, -C₁₋₆ substituted alkyl, -C₁₋₆ alkyl-Q₃, - C₁₋₆ alkyl-C₃₋₆ cycloalkyl-Q₃ and -C₁₋₆ substituted alkyl-Q₃;
R₁₆ is selected from the group of -H, -C₁₋₆ alkyl, -NR₂R₂, and -COOR₂;
with the proviso that when V is -NR₁₂-; R₁₆ is not -NR₂R₂; and
R₁₇ is selected from the group of -H, -C₁₋₆ alkyl, -COOR₃, and aryl.

Compounds of Formula II are described in WO 2017/134596 which is incorporated herein by reference. In an embodiment the maturation inhibitor is Compound 3. Compound 3 is described in WO 2017/134596 as Example 25 which example is incorporated herein by reference.

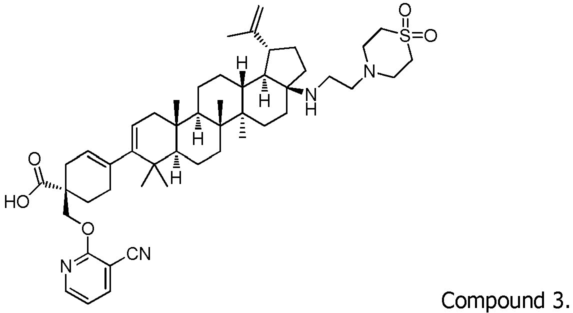

The pharmaceutical composition may be administered in combination with an NRTTI. In an embodiment the NRTTI is a compound of the formula 3: wherein: 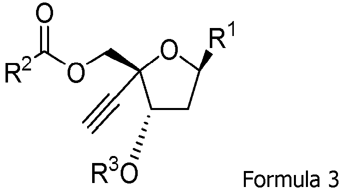
R¹ is:
wherein: 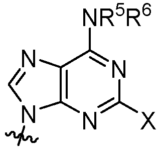
   X is selected from the group consisting of NH₂, F and Cl;
   R⁵ is selected from the group consisting of H and (C₁-C₁₄) alkyl;
   R⁶ is selected from the group consisting of H and -(C=O)-(C₁-C₁₄) alkyl;
   R² is selected from the group consisting of (C₁-C₂₄) alkyl; (CH₂)ₙ₁-O-(CH₂CH₂O)ₙ₂-(C₁-C₁₄ alkyl) where n1 and n2 are integers independently selected from 1-4; -R⁷-NH-(C=O)-R³ wherein R⁷ may be (C₁-C₁₄) alkyl and R⁸ may be independently selected from H and (C₁-C₁₄) alkyl; -R⁹-(C₆-C₁₄) aryl, wherein R⁹ is a bond or (C₁-C₆) alkyl; -R¹⁰-(C₃-C₁₄) cycloalkyl, wherein R¹⁰ is a bond or (C₁-C₆) alkyl; -(C₁-C₂₀) alkylene-(C=O)-O-R¹¹ wherein R¹¹ may be selected from H and (C₁-C₂₀)alkyl; 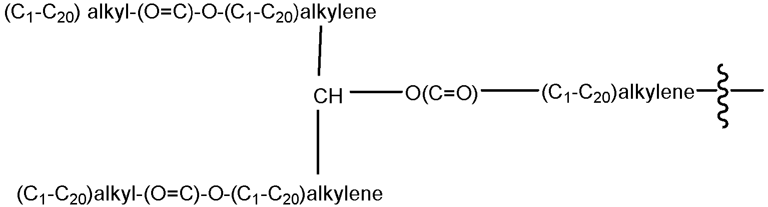 and;
   R³ is selected from the group consisting of H, -(C=O)-(C₁-C₂₄) alkyl; -(C=O)-O-(C₁-C₂₄) alkyl; and C₃-C₁₄ cycloalkyl; or
   R² and R³ join together to form a C₃ to C₂₈ cyclic structure; and
   with the proviso that when R² is (C₁-C₁₄ alkyl) at least one of R³, R⁵ and R⁶ is not H.

Compounds of Formula 3 are disclosed in WO 2020/178767 which is incorporated by reference herein. In an embodiment a pharmaceutical composition of the invention is combined with Compound 4. Compound 4 is described in WO 2020/178767 as Example 18, which example is incorporated by reference herein.

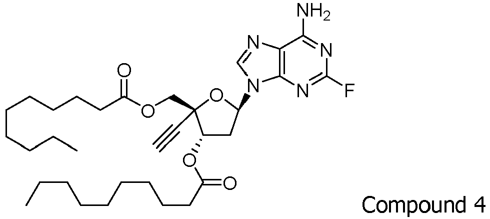

In an alternative embodiment the NRTTI is Islatravir.

In an embodiment of the invention the pharmaceutical compositions of the present invention are administered in combination with hyaluronidase. In one embodiment the pharmaceutical compositions of the present invention and the hyaluronidase are administered sequentially. The hyaluronidase may be administered subcutaneously or intramuscularly via injection. In one embodiment the hyaluronidase is administered prior to the administration of the pharmaceutical composition. In this embodiment the pharmaceutical composition is administered as close as possible to the site of the injection of hyaluronidase. It is thought that the hyaluronidase increases the permeability of the injection site tissues to the pharmaceutical compositions.

In a third aspect, the present invention provides a pharmaceutical composition defined herein for use in the treatment or prevention of HIV.

In one embodiment, the pharmaceutical composition is suitable for use as an injectable composition. In one embodiment the use comprises administering the pharmaceutical composition parenterally. In an embodiment the use comprises administering the pharmaceutical composition intramuscularly. In another embodiment the use comprises administering the pharmaceutical composition subcutaneously.

In an embodiment the use comprises administering around 1mL to around 3 mL of the pharmaceutical composition to a patient. In an embodiment the use comprises administering around 1mL of the pharmaceutical composition to a patient. In another embodiment the use comprises administering around 2 mL of the pharmaceutical composition to a patient. In an embodiment the use comprises administering around 3 mL of the pharmaceutical composition to a patient.

In an embodiment the use comprises administering the pharmaceutical to a patient once every 1, 2 or 3 months. In an embodiment the use comprises administering the pharmaceutical composition to a patient once every month. In an alternative embodiment, the use comprises administering the pharmaceutical composition once every 2 months. In an alternative embodiment the use comprises administering the pharmaceutical composition once every 3 months.

In one embodiment, the use comprises delivering the the pharmaceutical composition to a patient once every month. In this embodiment, the pharmaceutical composition may be administered by any suitable means. The pharmaceutical composition may be self-administered by a patient. In this embodiment the pharmaceutical composition may be administered subcutaneously via injection. The subcutaneous injection may comprise 1 to 1.5 mL of the pharmaceutical composition. In one embodiment of the invention the pharmaceutical composition is self-administered once monthly by subcutaneous injection.

In an alternative embodiment the use comprises delivering the pharmaceutical composition to a patient once every three months. In this embodiment, the pharmaceutical composition may be administered by any suitable means. The pharmaceutical composition may be administered intramuscularly via injection. In an embodiment the intramuscular injection is administered by a healthcare professional. The intramuscular injection may comprise 2.5 to 3 mL of the pharmaceutical composition. In an embodiment of the invention the pharmaceutical composition is administered once every 3 months by intramuscular injection.

In an embodiment of the invention use comprises administering the pharmaceutical compositions of the present invention in combination with other pharmaceutical compositions as a component of a multi drug treatment regimen. In an embodiment, the other pharmaceutical compositions are drugs which treat or prevent HIV. Marketed medicines are currently available to treat HIV.

In an embodiment the use comprises administering the pharmaceutical compositions of the present invention in combination with N6-LS. N6-LS is described above.

In an embodiment the use comprises administering the pharmaceutical compositions of the present invention in combination with a capsid inhibitor, a maturation inhibitor or a nucleoside reverse transcriptase translocation inhibitor (NRTTI).

The pharmaceutical composition may be administered in combination with a capsid inhibitor. In an embodiment the capsid inhibitor is a compound of Formula I, or a pharmaceutically acceptable salt thereof: 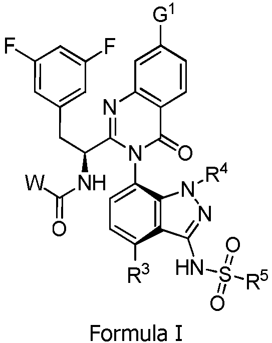 wherein:
G1 is phenyl substituted once with -N(CH3)S(O2)CH3, -S(O2)C(CH3)3, -CHF2, -CF3, -OCHF2, - OCF3, or -C(CH3)2OH, with the proviso that when G1 is -CHF2 or CF3, G1 is not in the para position or G1 is one of the following: 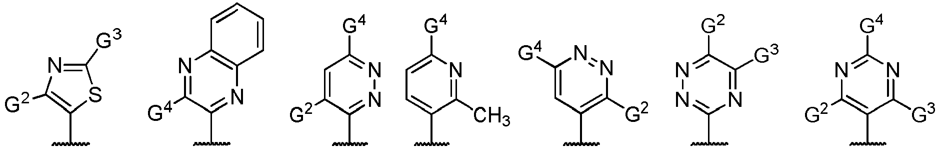 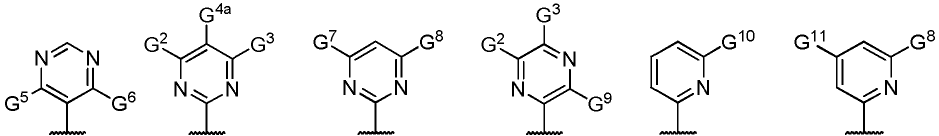 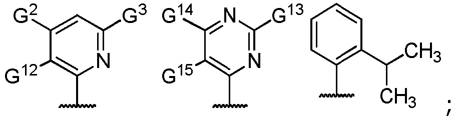
G² and G³ are independently selected from is H or -CH₃;
G⁴ is H, -CH₃, or -OCH₃;
G^{4a} is -CH₃, **or** -OCH₃;
G⁵ is -CH₃, or CH₂CH₃;
G⁶ is H, -CH₃, or CH₂CH₃;
G⁷ is ethyl, isopropyl, tert-butyl, -CHF₂, or -CF₃;
G⁸ is H, methyl, ethyl, -CHF₂, -CF₃, -OCH₃, or -OCH₂CH₃;
G⁹ is ethyl, isopropyl, cyclopropyl, -CH₂0H, -OCH₃;
G¹⁰ is ethyl, isopropyl, cyclopropyl, *tert*-butyl, -CHF₂, or -CF₃;
G¹¹ is methyl, -OCH₃, -CHF₂, -CF₃, -S(O₂)CH₃;
G¹² is F, -CH₃, -CHF₂, -CF₃, -OCH₃, -S(O₂)CH₃;
G¹³ is C₁-C₄alkyl, C₁-C₆cycloalkyl, -CH₂O(C₁-C₃alkyl);
G¹⁴ is H, C₁-C₄alkyl, -CHF₂, -CF₃, -O(C₁-C₃alkyl);
G¹⁵ is H, F, -CH₃, or OCH₃;
R³ is H, F, Cl, -CH₃, or -OCH₃;
R⁴ is H or C₁-C₃alkyl wherein C₁-C₃alkyl is optionally substituted with 1-3 fluorines;
R⁵ is C₁-C₆ alkyl or C₃-C₆ cycloalkyl;
W is selected from: 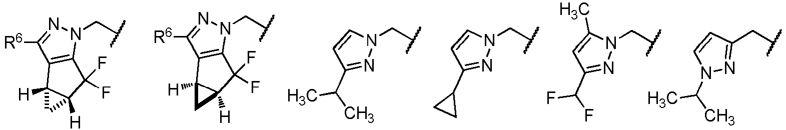 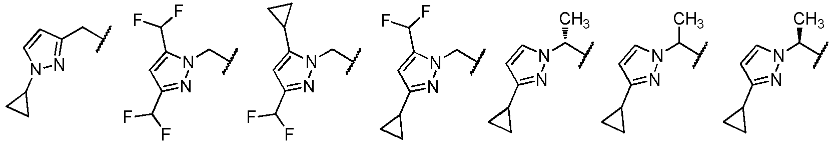 where R⁶ is methyl optionally substituted with 1 to 3 fluorines.

Compounds of Formula I are described in WO2020/084492 which is incorporated by reference. In an embodiment the capsid inhibitor is Compound 1 or a pharmaceutically acceptable salt thereof. Compound 1 is described in WO 2020/084492 as Example 59 which example is incorporated herein by reference.

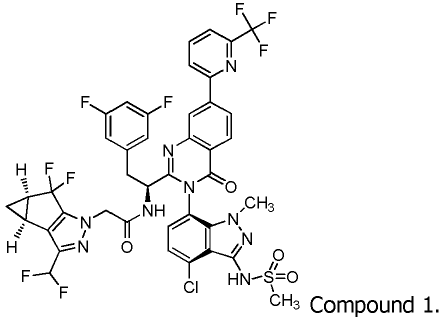

In an embodiment the capsid inhibitor is Compound 2 pharmaceutically acceptable salt thereof. Compound 2 is described in patent application number PCT/IB2020/055653 as Example 1, which example is incorporated herein by reference.

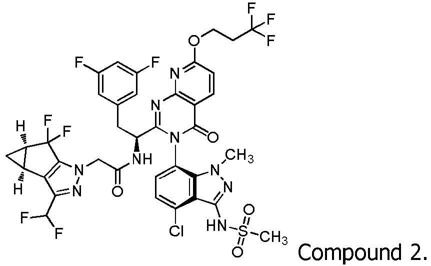

In an alternative embodiment the capsid inhibitor is lenacapravir.

The pharmaceutical composition may be administered in combination with a maturation inhibitor. In an embodiment the maturation inhibitor is a compound of Formula II or a pharmaceutically acceptable salt thereof: 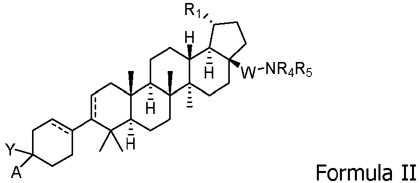
wherein R₁ is isopropenyl or isopropyl;
A is -C₁₋₆ alkyl-OR₀;
wherein R₀ is heteroaryl-Q₀;
Q₀ is selected from the group of -H, -CN, -C₁₋₆ alkyl, -COOH, -Ph, -OC₁₋₆ alkyl, -halo, -CF₃,
Y is selected from the group of -COOR₂, -C(O)NR₂SO₂R₃, -C(O)NHSO₂NR₂R₂, - SO₂NR₂C(O)R₂, -tetrazole, and -CONHOH,
wherein n = 1-6;
R₂ is -H, -C₁₋₆ alkyl, -alkylsubstituted C₁₋₆ alkyl or-arylsubstituted C₁₋₆ alkyl;
W is absent, or is -CH₂- or -CO-;
R₃ is -H, -C₁₋₆ alkyl or -alkylsubstituted C₁₋₆ alkyl;
R₄ is selected from the group of -H, -C₁₋₆ alkyl, -C₁₋₆ alkyl-C₃₋₆ cycloalkyl, -C₁₋₆ substituted -C₁₋₆ alkyl, -C₁₋₆ alkyl-Q₁, -C₁₋₆ alkyl-C₃₋₆ cycloalkyl-Q₁, aryl, heteroaryl, substituted heteroaryl, -COR₆, - SO₂R₇, -SO₂NR₂R₂, and 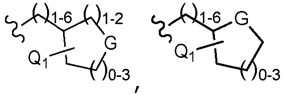
wherein G is selected from the group of -0-, -SO₂- and -NR₁₂-;
wherein Q₁ is selected from the group of -C₁₋₆ alkyl, - C₁₋₆ fluoroalkyl, heteroaryl, substituted heteroaryl, halogen, -CF₃, -OR₂, -COOR₂, -NR₈R₉, -CONR₈R₉ and -SO₂R₇;
R₅ is selected from the group of -H, -C₁₋₆ alkyl, -C₃₋₆ cycloalkyl, -C₁₋₆ alkylsubstituted alkyl, -C₁₋₆ alkyl-NR₈R₉, -COR₃, -SO₂R₇ and -SO₂NR₂R₂;
with the proviso that R₄ or R₅ is not -COR₆ when W is -CO-;
with the further proviso that only one of R₄ or R₅ is selected from the group of -COR₆, -COCOR₆,-SO₂R₇ and -SO₂NR₂R₂;
R₆ is selected from the group of -H, -C₁₋₆ alkyl, -C₁₋₆ alkyl-substitutedalkyl, -C₃₋₆ cycloalkyl, -C₃₋₆ substitutedcycloalkyl-Q₂, -C₁₋₆ alkyl-Q₂, -C₁₋₆ alkyl-substitutedalkyl-Q₂,-C₃₋₆ cycloalkyl-Q₂, aryl-Q₂, - NR₁₃R₁₄, and -OR₁₅;
wherein Q₂ is selected from the group of aryl, heteroaryl, substituted heteroaryl, -OR₂, -COOR₂, - NR₈R₉, SO₂R₇, -CONHSO₂R₃, and -CONHSO₂NR₂R₂;
R₇ is selected from the group of -H, -C₁₋₆ alkyl, -C₁₋₆ substituted alkyl, -C₃₋₆ cycloalkyl, -CF₃, aryl, and heteroaryl;
R₈ and R₉ are independently selected from the group of -H, -C₁₋₆ alkyl, -C₁₋₆ substituted alkyl, aryl, heteroaryl, substituted aryl, substituted heteroaryl, -C₁₋₆ alkyl-Q₂, and -COOR₃, or R₈ and R₉ are taken together with the adjacent N to form a cycle selected from the group of: 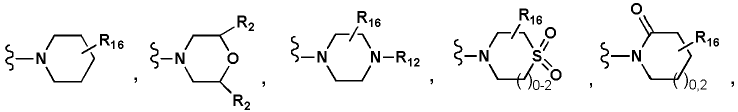 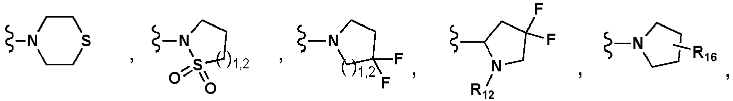 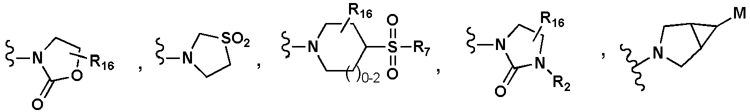 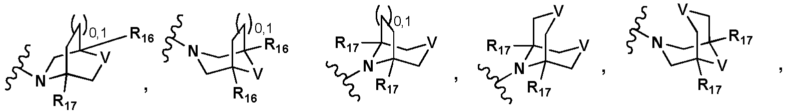 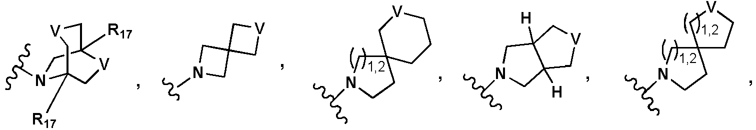 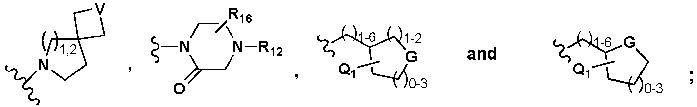
M is selected from the group of -R₁₅, -SO₂R₂, -SO₂NR₂R₂, -OH and -NR₂R₁₂;
V is selected from the group of -CR₁₀R₁₁-, -SO₂-, -O- and -NR₁₂-;
with the proviso that only one of R₈ or R₉ can be -COOR₃;
R₁₀ and R₁₁ are independently selected from the group of -H, -C₁₋₆ alkyl, -C₁₋₆ substituted alkyl and -C₃₋₆ cycloalkyl;
R₁₂ is selected from the group of -H, -C₁₋₆ alkyl, -alkylsubstituted C₁₋₆ alkyl, -CONR₂R₂, -SO₂R₃, and -SO₂NR₂R₂;
R₁₃ and R₁₄ are independently selected from the group of -H, -C₁₋₆ alkyl, -C₃₋₆ cycloalkyl, -C₁₋₆ substituted alkyl, -C₁₋₆ alkyl-Q₃, -C₁₋₆ alkyl-C₃₋₆ cycloalkyl-Q₃, and C₁₋₆ substituted alkyl-Q₃;
Q₃ is selected from the group of heteroaryl, substituted heteroaryl, -NR₂R₁₂, -CONR₂R₂, -COOR₂, - OR₂, and -SO₂R₃;
R₁₅ is selected from the group of -C₁₋₆ alkyl, -C₃₋₆ cycloalkyl, -C₁₋₆ substituted alkyl, -C₁₋₆ alkyl-Q₃, - C₁₋₆ alkyl-C₃₋₆ cycloalkyl-Q₃ and -C₁₋₆ substituted alkyl-Q₃;
R₁₆ is selected from the group of -H, -C₁₋₆ alkyl, -NR₂R₂, and -COOR₂;
with the proviso that when V is -NR₁₂-; R₁₆ is not -NR₂R₂; and
R₁₇ is selected from the group of -H, -C₁₋₆ alkyl, -COOR₃, and aryl.

Compounds of Formula II are described in WO 2017/134596 which is incorporated herein by reference. In an embodiment the maturation inhibitor is Compound 3. Compound 3 is described in WO 2017/134596 as Example 25 which example is incorporated herein by reference.

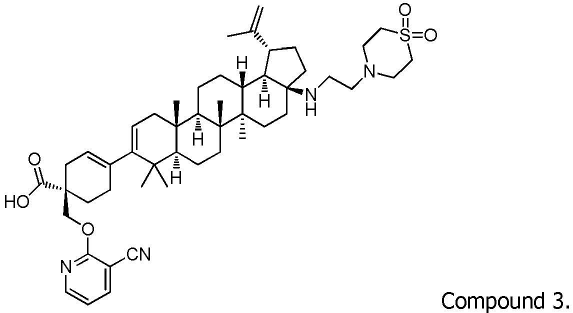

The pharmaceutical composition may be administered in combination with an NRTTI. In an embodiment the NRTTI is a compound of the formula 3: 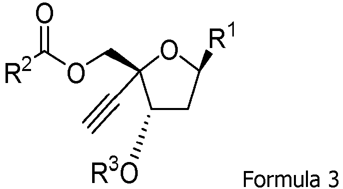 wherein:
R¹ is: 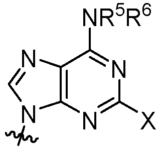
wherein:
   X is selected from the group consisting of NH₂, F and Cl;
   R⁵ is selected from the group consisting of H and (C₁-C₁₄) alkyl;
   R⁶ is selected from the group consisting of H and -(C=O)-(C₁-C₁₄) alkyl;
   R² is selected from the group consisting of (C₁-C₂₄) alkyl; (CH₂)ₙ₁-O-(CH₂CH₂O)ₙ₂-(C₁-C₁₄ alkyl) where n1 and n2 are integers independently selected from 1-4; -R⁷-NH-(C=O)-R⁸ wherein R⁷ may be (C₁-C₁₄) alkyl and R⁸ may be independently selected from H and (C₁-C₁₄) alkyl; -R⁹-(C₆-C₁₄) aryl, wherein R⁹ is a bond or (C₁-C₆) alkyl; -R¹⁰-(C₃-C₁₄) cycloalkyl, wherein R¹⁰ is a bond or (C₁-C₆) alkyl; -(C₁-C₂₀) alkylene-(C=O)-O-R¹¹ wherein R¹¹ may be selected from H and (C₁-C₂₀)alkyl; 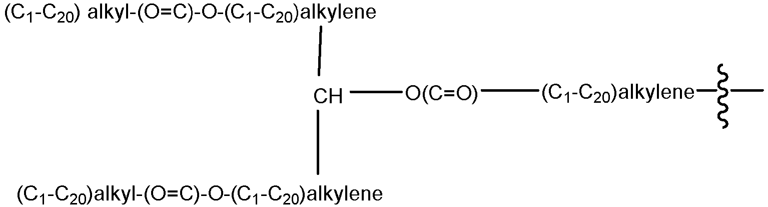 and;
   R³ is selected from the group consisting of H, -(C=O)-(C₁-C₂₄) alkyl; -(C=O)-O-(C₁-C₂₄) alkyl; and C₃-C₁₄ cycloalkyl; or
   R² and R³ join together to form a C₃ to C₂₈ cyclic structure; and
   with the proviso that when R² is (C₁-C₁₄ alkyl) at least one of R³, R⁵ and R⁶ is not H.

Compounds of Formula 3 are disclosed in WO 2020/178767 which is incorporated by reference herein. In an embodiment a pharmaceutical composition of the invention is combined with Compound 4. Compound 4 is described in WO 2020/178767 as Example 18, which example is incorporated by reference herein.

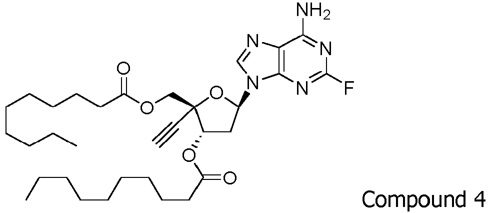

In an alternative embodiment the NRTTI is Islatravir.

Further disclosed herein is a kit comprising cabotegravir or a pharmaceutically acceptable salt thereof, polyethylene glycol and poloxamer. Disclosed herein, the kit comprises a syringe comprising a composition of the invention as well as a leaflet comprising use instructions.

The following non-limiting Examples illustrate the present invention.

### EXAMPLES

### Example 1: Composition of the invention

**Table 3**

| **Ingredient** | **Concentration (mg/mL)** |
|---|---|
| Cabotegravir | 400.0 |
| Poloxamer 338 | 38.0 |
| Polyethylene Glycol 3350 | 35.0 |
| Mannitol | 20.0 |

Table 3 shows an exemplary pharmaceutical composition of the invention (pharmaceutical compositions are also described in these examples as "suspensions") which was made using the following method.

A formulation vehicle was prepared by dissolving 9.5g Poloxamer 338 (BASF), 5.0g mannitol (Roquette Freres), and 8.7g PEG3350 (Clariant) in 164.2g water for injection (WFI) and filtering the solution through a 0.2µm filter. The formulation vehicle was added to cabotegravir (free acid) to prepare a 400 mg/ml coarse suspension. The coarse suspension, while stirred, was circulated through a wet bead mill set at 29.7Hz (Netzsch MiniCer) containing 0.30mm YTZ grinding beads (Nikkato Corp) at 73 - 145 ml/min until the desired median particle diameter of 0.2 to 1.0 µm was reached as measured by laser diffraction. The wet bead mill was cooled to maintain a temperature between 1 and 25°C. The resulting suspension was filled into Type I glass vials, flushed with nitrogen, stoppered (FM457 stopper) and sealed. The suspension was terminally sterilized by gamma irradiation at a minimum dose of 25kGy.

### Example 2: Stability of a composition comprising Cabotegravir, Poloxamer 338 and PEG3350

A formulation vehicle was prepared by dissolving 7.5g Poloxamer 338 (BASF), 5.7g mannitol (Roquette Freres), and 7.5g PEG3350 (Clariant) in 166.7g water for injection (WFI) and filtering the solution through a 0.2µm filter. The formulation vehicle was added to 100g Cabotegravir (free acid) to prepare a 400 mg/ml coarse suspension. The coarse suspension, while stirred, was circulated through a wet bead mill set at 29.7Hz (Netzsch MiniCer) containing 0.30mm YTZ grinding beads (Nikkato Corp) at 73 - 145 ml/min until a desired median particle diameter of 0.25µm was reached. The wet bead mill was cooled to maintain a temperature between 1 and 25°C. The milled suspension was filled into Type I glass vials, flushed with nitrogen, stoppered (FM457 stopper) and sealed. The resulting suspension was terminally sterilized by gamma irradiation at a minimum dose of 25kGy. Table 4 shows the suspension which was made.

**Table 4**

| | | | | |
|---|---|---|---|---|
| **Suspension 1** | 398 mg/mL cabotegravir (measured by HPLC) | 30 mg/mL P338 | 30 mg/mL PEG3350 | 23 mg/mL mannitol |

Suspension 1 was stored at ambient laboratory conditions for 3 months and then evaluated for resuspension using an automated vial shaker. Assessment of the time it took the suspension to resuspend was performed by using an automatic shaker to reduce variability in shaking (Glas-Col Benchtop Shaker). The vial comprising the composition was placed into a holder on the shaker and was shaken at 230 to 350 motor speed for 30 second intervals until the bottom of the vial was assessed through visual inspection to be clear of sedimented/caked suspension material. Figure 1 shows a micrograph of the bottom of a vial which contained Suspension 1 after 30 seconds of shaking by the automated shaker. Based on the microscopy images, Suspension 1 (left image in Figure 1) was superior in resuspension compared to Suspension 2 which is the same formulation, made using the method described above, but comprising Kollidon 12 (polyvinylpyrrolidone) instead of PEG3350. Suspension 1 (left image in Figure 1) shows a mostly clear vial bottom indicating that the suspension was resuspended and decanted. Suspension 2 shows a vial bottom with suspension (*i*.*e*. crystalline cabotegravir particles) adhered to the bottom of the vial, meaning that resuspension was not achieved. Both formulations were shaken using the automated shaker for 30 seconds, but Suspension 2 was hand shaken to try to further resuspend the product.

Particle size of Suspension 1 was measured after vials were stored in an upright position at accelerated (40°C/75%RH) and stressed (50°C/ambient) stability conditions. Particle size analysis for the particles in the suspension was performed using laser diffraction (Malvern Mastersizer 3000). This method generates particle size distributions for the samples. The instrument uses a proprietary algorithm to convert the 2-dimensional scattering pattern generated by the interaction of the laser with particles in the sample to a particle size distribution; the scattering pattern is dependent on the size distribution of the particles in the sample. Table 6 explains the definitions used reporting particle size distributions.

**Table 5**

| | Initial particle size | | | 1 month 40°C/75%RH | | | 1 month 50°C/ambient | | |
|---|---|---|---|---|---|---|---|---|---|
| | **x10** | **x50** | **x90** | **x10** | **x50** | **x90** | **x10** | **x50** | **x90** |
| Suspension 1 | 0.07 | 0.15 | 0.37 | 0.07 | 0.16 | 0.39 | 0.08 | 0.18 | 0.45 |

**Table 6**

| | |
|---|---|
| x50 | Median particle diameter, µm; here used on a volumetric basis, i.e. 50% by volume of the particles is smaller than this diameter and 50% is larger |
| x10 | Particle diameter corresponding to 10% of the cumulative undersize distribution (here by volume), µm |
| x90 | Particle diameter corresponding to 90% of the cumulative undersize distribution (here by volume), µm |

Table 5 shows minimal change in particle size of Suspension 1 over time and at varying conditions indicating stability of the suspension.

### Example 3 (reference example): 400 mg/mL Cabotegravir with Poloxamer 338 or 407

Injectable suspensions comprising about 400 mg/mL cabotegravir were prepared with Poloxamer 407 or 338. Mannitol was also added as a tonicity agent. The formulation vehicle was prepared by dissolving: 7.0g Poloxamer 338 and 3.5g mannitol in 177.0 g WFI (Suspension 4) or 7.5g P407 (BASF) and 4.7g mannitol (Roquette Freres) in 175.3g WFI (Suspension 3) and filtering the solution through a 0.2µm filter. The formulation vehicle was added to 100g Cabotegravir (free acid) to prepare a 400 mg/ml coarse suspension. The coarse suspension, while stirred, was circulated through a wet bead mill set at 29.7Hz (Netzsch MiniCer) containing 0.30mm YTZ grinding beads (Nikkato Corp) at 73 - 145 ml/min until the desired mean particle diameter of around 0.2µm was reached. The wet bead mill was cooled to maintain a temperature between 1 to 25°C. The suspension was filled into Type I glass vials, flushed with nitrogen, stoppered (FM457 stopper) and sealed. The resulting suspension was terminally sterilized by gamma irradiation at a minimum dose of 25kGy.

Table 7 shows the suspensions which were made. Each suspension was stored at 40°C/75%RH in an upright position. Initial particle size and particle size after 1 month was measured as an indicator of suspension stability. Table 8 shows the particle size in each suspension after 1 month. There was no change between initial particle size and particle size after 1 month. The lack of change in particle size is an indicator of stability of the suspensions.

**Table 7**

| | | | |
|---|---|---|---|
| Suspension 3 | 389 mg/mL cabotegravir (measured by HPLC) | 30 mg/mL P407 | 19 mg/mL mannitol |
| Suspension 4 | 393 mg/mL cabotegravir (measured by HPLC) | 28 mg/mL P338 | 14 mg/mL mannitol |

**Table 8**

| Suspension | **Initial** | | | **1 month** | | |
|---|---|---|---|---|---|---|
| | X10 (um) | X50 (um) | X90 (um) | X10 (um) | X50 (um) | X90 (um) |
| Suspension 3 | 0.059 | 0.137 | 0.338 | 0.06 | 0.14 | 0.35 |
| Suspension 4 | 0.12 | 0.28 | 0.74 | 0.12 | 0.28 | 0.75 |

### Example 4: Ability of compositions comprising Cabotegravir, Poloxamer 338 and PEG3350 to resuspend

Sixteen suspensions were made varying levels of PEG3350, p338 and particle size as shown in Table 9. All formulations were prepared using the above described methods i.e. by dissolving Poloxamer 338 (BASF), mannitol (Roquette Freres), and PEG3350 (Clariant) in water for injection (WFI) and filtering the solution through a 0.2µm filter. The formulation vehicle was added to cabotegravir (free acid) to prepare an about 400 mg/ml coarse suspension. The coarse suspension, while stirred, was circulated through a wet bead mill set at 29.7Hz (Netzsch MiniCer) containing 0.30mm YTZ grinding beads (Nikkato Corp) at 73 - 145 ml/min until desired mean particle diameter of 0.2, 0.6 or 1.0 µm was reached as measured by laser diffraction. The wet bead mill was cooled to maintain a temperature between 1 to 25°C. The suspension was filled into Type I glass vials, flushed with nitrogen, stoppered (FM457 stopper) and sealed. The filled suspension was terminally sterilized by gamma irradiation at a minimum dose of 25kGy. The formulation was selected based on time to resuspension, particle size stability, viscosity and injection force. All suspensions made comprised about 400 mg/mL cabotegravir and about 30 mg/mL mannitol. The concentrations of P338 and PEG338 were varied. The suspensions made are listed below in Table 9.

**Table 9**

| **Suspension** | **Cabotegravir (mg/mL) by HPLC** | **pH (duplicate measurements)** | **P338 (mg/mL)** | **PEG3350 (mg/mL)** | **Target x50 (µm)** |
|---|---|---|---|---|---|
| 1a | 402 | 4.6, 4.5 | 26 | 20 | 0.2 |
| 2a | 404 | 4.3, 4.5 | 26 | 50 | 0.2 |
| 3a | 405 | 5.2, 5.3 | 38 | 35 | 0.2 |
| 4a | 396 | 4.4, 4.1 | 50 | 0 | 0.2 |
| 5a | 406 | 4.3, 4.3 | 50 | 20 | 0.2 |
| 6a | 406 | 4.8, 4.8 | 50 | 50 | 0.2 |
| 7a | 403 | 4.9, 4.8 | 26 | 35 | 0.6 |
| 8a | 397 | 4.8, 4.7 | 38 | 20 | 0.6 |
| 9a | 394 | 4.0, 4.3 | 38 | 35 | 0.6 |
| 10a | 401 | 5.0, 4.9 | 38 | 50 | 0.6 |
| 11a | 398 | 5.1, 4.7 | 50 | 35 | 0.6 |
| 12a | 401 | 4.4, 4.6 | 26 | 20 | 1.0 |
| 13a | 402 | 5.0, 5.1 | 26 | 50 | 1.0 |
| 14a | 400 | 4.9, 4.8 | 38 | 35 | 1.0 |
| 15a | 402 | 4.1,4.4 | 50 | 20 | 1.0 |
| 16a | 402 | 5.0, 5.2 | 50 | 50 | 1.0 |

Each suspension was tested for its ability to resuspend. Vials were stored in an upright position prior to testing. The vials were then inserted into the holder of a benchtop shaker (Glas-Col) so that the vials were at an angle of approximately 45° (1 and 2 months) to the ground with the top of the vial facing downwards, or 0° (3 and 6 months) to the ground. The vials were shaken for 30 seconds at a motor speed setting of 230 to 250. After shaking, the samples were visually inspected to see if the suspension had resuspended. Resuspension was determined visually as absence of product caked to the bottom of the vial. If the bottom of the vial was transparent to light, it was determined that the suspension had resuspended. If the product had not yet resuspended, it was shaken for another 30 seconds and the bottom of the vial was checked again. Shaking and visual inspection were continued until the product visually appeared resuspended after a maximum of 4 shaking increments (up to 3 months) or a maximum of 5 shaking increments at 6 months.

The shaking angle of the test was changed from 45° to 0° after 2 months to improve resuspension because the majority of suspensions did not resuspend after 2 months at 30°C/65%RH. The 45° angle is not as representative of human shaking as the 0° angle when Instruction for Use (IFU) instructions for suspension redispersion prior to administration are followed. At 2 months, samples were noted with an asterisk if the vials were not fully resuspended by visual observation. At 3 and 6 months, microscope images were used to determine whether product was fully resuspended; samples containing residual product on the vial bottom were noted with an asterisk. An example of suspensions considered to be fully resuspended (A) and not resuspended (B) are in Figure 4.

Table 10 shows resuspension data after 2 months upright storage at 30°C/65%RH. The number of 30-second shaking intervals to resuspend 3 vials per suspension is shown. The automated shaker was used to reduce the variability in force and torque experienced by the vial during shaking.

Suspension 3a required the lowest number of shaking intervals to resuspend the vials. The three suspensions which required the least number of shaking intervals were all 0.2 µm formulations indicating that this particle size allows for good resuspension.

Table 11 shows resuspension data after 3 and 6 months upright storage at 30°C/65%RH. The number of 30-second shaking intervals to resuspend 3 vials per suspension is shown. Suspension 3a continued to require a lower number of 30 sec increments of shaking on the benchtop shaker per vial for product resuspension after 6 months compared to most of the other formulations.

**Table 10 - *indicates that suspensions were not fully resuspended after 4 shakes per vial**

| **Suspension** | **P338 (mg/mL)** | **PEG3350 (mg/mL)** | **Target Size (µm)** | **# of 30 second shaking intervals (3 replicates)** | | | **Total** |
|---|---|---|---|---|---|---|---|
| 3a | 38 | 35 | 0.2 | 2 | 2 | 2 | 6 |
| 4a | 50 | 0 | 0.2 | 3 | 2 | 2 | 7 |
| 1a | 26 | 20 | 0.2 | 2 | 3 | 3 | 8 |
| 6a | 50 | 50 | 0.2 | 3 | 3 | 3 | 9 |
| 9a | 38 | 35 | 0.6 | 4 | 4 | 4 | 12 |
| 16a | 50 | 50 | 1 | 4 | 4 | 4 | 12 |
| 2a | 26 | 50 | 0.2 | 4 | 4 | 4 | *12 |
| 5a | 50 | 20 | 0.2 | 4 | 4 | 4 | *12 |
| 11a | 50 | 35 | 0.6 | 4 | 4 | 4 | *12 |
| 14a | 38 | 35 | 1 | 4 | 4 | 4 | *12 |
| 13a | 26 | 50 | 1 | 4 | 4 | 4 | *12 |
| 15a | 50 | 20 | 1 | 4 | 4 | 4 | *12 |
| 12a | 26 | 20 | 1 | 4 | 4 | 4 | *12 |
| 8a | 38 | 20 | 0.6 | 4 | 4 | 4 | *12 |
| 7a | 26 | 35 | 0.6 | 4 | 4 | 4 | *12 |
| 10a | 38 | 50 | 0.6 | 4 | 4 | 4 | *12 |

**Table 11 - * indicates that suspensions were not fully resuspended after 4 or 5 shakes per vial via evaluation using a light microscope. ^indicates that only 2 vials were tested. 3 vials were tested for all other conditions.**

| | 3 months 30°C/65%RH | | 6 months 30°C/65%RH | |
|---|---|---|---|---|
| Suspension | Total | Avg/Vial | Total | Avg/Vial |
| 1a | 5 | 1.7 | 6 | 2.0 |
| 2a | 12 | 4.0* | 15 | 5.0* |
| 3a | 5 | 1.7 | 6 | 2.0 |
| 4a | 3 | 1.0 | 3 | 1.0 |
| 5a | 6 | 2.0 | 6^ | 3.0 |
| 6a | 7 | 2.3 | 9 | 3.0 |
| 7a | 6 | 2.0 | 7 | 2.3 |
| 8a | 6 | 2.0 | 7 | 2.3 |
| 9a | 8 | 2.7 | 15 | 5.0 |
| 10a | 6 | 2.0 | 15 | 5.0 |
| 11a | 6 | 2.0 | 15 | 5.0 |
| 12a | 12 | 4.0* | 15 | 5.0* |
| 13a | 9 | 3.0 | 15 | 5.0 |
| 14a | 5 | 1.7 | 6 | 2.0 |
| 15a | 3 | 1.0 | 6 | 2.0 |
| 16a | 6 | 2.0 | 14 | 4.7 |

### Example 5: Particle size and viscosity of compositions comprising Cabotegravir, Poloxamer 338 and PEG3350

The viscosity of each of the suspensions made in Table 9 in Example 4 was measured initially after they were made. The particle size of the suspensions was also measured initially after they were made as well as after 2, 3 and 6 months of storage at 40°C/75%RH. Results are shown in Table 12 and 13. Across all suspensions, there was minimal change in particle size after storage under accelerated conditions. Viscosity differed across suspensions. Suspensions with a target x50 of 0.2µm and 50 mg/mL P338 had the highest viscosities. The shear viscosity of the suspensions was measured using a Malvern Kinexus Pro rheometer equipped with a roughened cone and plate. Data was gathered at 20 °C with data collection at 10 points per decade across a shear rate range from 0.1 s-1 to 10 s-1.

**Table 12**

| | | | Initial | | | 2 MN 40°C/75%RH | | |
|---|---|---|---|---|---|---|---|---|
| **Suspensions** | **Visc** @ **0.1 s⁻¹ (cP)** | **Visc** @ **10 s⁻¹ (cP)** | **X10 (µm)** | **X50 (µm)** | **X90 (µm)** | **X10 (µm)** | **X50 (µm)** | **X90 (µm)** |
| 1a | 24 | 18 | 0.09 | 0.2 | 0.5 | 0.11 | 0.23 | 0.54 |
| 2a | 38 | 24 | 0.1 | 0.23 | 0.54 | 0.12 | 0.26 | 0.59 |
| 3a | 116 | 43 | 0.07 | 0.17 | 0.39 | 0.08 | 0.17 | 0.4 |
| 4a | 406 | 76 | 0.09 | 0.21 | 0.59 | 0.1 | 0.24 | 0.79 |
| 5a | 763 | 95 | 0.1 | 0.22 | 0.55 | 0.73 | 0.24 | 0.58 |
| 6a | 392 | 79 | 0.09 | 0.2 | 0.51 | 0.11 | 0.24 | 0.57 |
| 7a | 83 | 25 | 0.21 | 0.6 | 1.60 | 0.28 | 0.70 | 2.0 |
| 8a | 50 | 28 | 0.23 | 0.66 | 1.90 | 0.27 | 0.71 | 2.1 |
| 9a | 92 | 31 | 0.18 | 0.61 | 1.7 | 0.26 | 0.68 | 2.0 |
| 10a | 132 | 40 | 0.23 | 0.66 | 1.90 | 0.34 | 0.76 | 2.3 |
| 11a | 124 | 44 | 0.24 | 0.69 | 2.00 | 0.33 | 0.78 | 2.3 |
| 12a | 17 | 11 | 0.33 | 0.96 | 2.6 | 0.43 | 1.0 | 2.7 |
| 13a | 32 | 16 | 0.57 | 1.3 | 3.0 | 0.6 | 1.4 | 3.1 |
| 14a | 54 | 24 | 0.49 | 1.2 | 3.0 | 0.52 | 1.2 | 3.0 |
| 15a | 37 | 15 | 0.74 | 1.6 | 3.3 | 0.73 | 1.6 | 3.4 |
| 16a | 92 | 55 | 0.61 | 1.4 | 3.1 | 0.6 | 1.4 | 3.2 |

**Table 13 - No major changes observed in particle size after 6 months at 40°C/75%RH.**

| | **3 months 40°C/75%RH** | | | **6 months 40°C/75%RH** | | |
|---|---|---|---|---|---|---|
| Suspension | x10 (µm) | x50 (µm) | x90 (µm) | x10 (µm) | x50 (µm) | x90 (µm) |
| 1a | 0.1 | 0.22 | 0.53 | 0.09 | 0.21 | 0.57 |
| 2a | 0.11 | 0.26 | 0.62 | 0.10 | 0.24 | 0.63 |
| 3a | 0.07 | 0.16 | 0.4 | 0.07 | 0.16 | 0.4 |
| 4a | 0.08 | 0.22 | 0.83 | 0.09 | 0.23 | 0.8 |
| 5a | 0.08 | 0.2 | 0.54 | 0.09 | 0.21 | 0.53 |
| 6a | 0.1 | 0.23 | 0.58 | 0.09 | 0.24 | 0.71 |
| 7a | 0.16 | 0.63 | 1.7 | 0.19 | 0.67 | 1.9 |
| 8a | 0.15 | 0.67 | 1.8 | 0.21 | 0.67 | 1.9 |
| 9a | 0.22 | 0.67 | 2.0 | 0.20 | 0.66 | 1.9 |
| 10a | 0.19 | 0.69 | 2.0 | 0.21 | 0.73 | 2.0 |
| 11a | 0.18 | 0.73 | 2.1 | 0.21 | 0.77 | 2.1 |
| 12a | 0.41 | 1.0 | 2.7 | 0.35 | 0.99 | 2.6 |
| 13a | 0.6 | 1.4 | 3.1 | 0.6 | 1.4 | 3.1 |
| 14a | 0.52 | 1.2 | 3.0 | 0.54 | 1.3 | 3.0 |
| 15a | 0.71 | 1.6 | 3.3 | 0.69 | 1.6 | 3.3 |
| 16a | 0.6 | 1.4 | 3.2 | 0.6 | 1.4 | 3.2 |

### Example 6: 12-month stability of suspension 6a

A suspension with the P338 and PEG3350 composition and target size of suspension 6a was manufactured for a non-clinical safety study and monitored on stability for 12 months. The data is shown in Table 14. No changes in content, impurities or particle size were observed. A drop in pH was observed, but the pH is still within the acceptable pH range for injection.

**Table 14**

| **Storage Condition/ Orientation** | **Storage Time (Months)** | **Cabotegravir Content by HPLC (mg/mL)** | **Drug Related Impurities (% area)** | **pH** | **Particle Size (µm)** | | |
|---|---|---|---|---|---|---|---|
| | | | | | **X₁₀** | **X₅₀** | **X₉₀** |
| Initial | Initial | 398.8 | 0.05 | 5.6, 5.5 | 0.05 | 0.12 | 0.28 |
| 30°C/65%RH /Inverted | 2 | 400.0 | 0.05 | 4.8, 4.8 | 0.05 | 0.14 | 0.36 |
| | 3 | 401.2 | 0.05 | 4.8, 4.6 | 0.05 | 0.13 | 0.29 |
| | 6 | 398.0 | 0.05 | 4.6, 4.7 | 0.06 | 0.14 | 0.31 |
| | 9 | 401.6 | 0.05 | 4.5, 4.7 | 0.06 | 0.13 | 0.31 |
| | 12 | 396.0 | 0.05 | 4.5, 4.7 | 0.05 | 0.12 | 0.29 |

### Example 7: Pharmacokinetics

The pharmacokinetics of cabotegravir suspensions were evaluated in male Sprague Dawley rats. Three suspensions were tested: (i) 200 mg/mL cabotegravir with PS20 and PEG3350; (ii) 400 mg/mL cabotegravir with P338; (iii) and 400 mg/mL cabotegravir with P338 and PEG3350; suspensions (i) and (ii) are reference compositions. The suspensions tested are shown in Table 15.

**Table 15**

| Suspension | Cabotegravir (mg/mL) by HPLC | Excipients | Size (x10) in µm | Size (x50) in µm | Size (x90) in µm |
|---|---|---|---|---|---|
| i | 195 | 18 mg/mL PS20, 18 mg/mL PEG3350, 31 mg/mL mannitol | 0.07 | 0.17 | 0.51 |
| ii | 393 | 28 mg/mL P338, 14 mg/mL mannitol | 0.12 | 0.28 | 0.74 |
| iii | 399 | 50 mg/mL P338, 50 mg/mL PEG3350, 30 mg/mL mannitol | 0.05 | 0.12 | 0.28 |

Rats were administered the cabotegravir suspensions by intramuscular injection in the right gastrocnemius muscle or subcutaneous injection at 200 or 400 mL/kg (rats weighed just prior to dosing) to achieve a target dose of 10 mg/kg/dose. A total of three rats were used per suspension. Blood samples were collected into 500 uL K₂EDTA tubes via lateral tail vein or tail tip amputation. 75 uL of whole blood was then transferred to a new matrix tube, combined with 75 uL of sterile water, vortexed and stored on dry ice. Samples were stored at -80°C for drug concentration analysis. Blood concentrations of cabotegravir were determined using an analytical method based on protein precipitation, followed by UHPLC/MS/MS analysis. Using a 25 µL aliquot of sample, the lower limit of quantification (LLQ) for cabotegravir was 50 ng/mL. The computer systems that were used on this study to acquire and quantify data included Analyst Version 1.6.1 and SMS2000 Version 3.1.

Figure 2 shows cabotegravir concentration in blood of individual rats after intramuscular administration of suspension i, ii or iii. Overall, the pharmacokinetic profiles for all three suspensions were found to be simiar. In particular, the slopes for the terminal phase, representing absorption rates for long acting formulations under the assumption of 'flip-flop kinetics', overlap for all formulations tested. This suggests that the long-acting profile for cabotegravir is maintained for the 400 mg/mL suspensions compared to the 200 mg/mL suspension.

Figure 3 shows cabotegravir concentrations in blood after a 10 mg/kg subcutaneous administration of 400 mg/mL cabotegravir with 50 mg/mL P338, 50 mg/mL PEG3350 and 30 mg/mL mannitol. 3 rats were used. Each line represents the blood concentration of a separate rat. Subcutaneous administration resulted in detectable blood cabotegravir concentrations out to 12 weeks.

### Example 8 (reference example): Comparison with a suspension comprising 400 mg/mL Cabotegravir with Polysorbate 20 (PS20) and Polyethylene Glycol 3350 (PEG3350)

A long acting suspension comprising 200 mg/mL cabotegravir, 20 mg/mL PS20 and 20 mg/mL PEG3350 was investigated to test stability when the cabotegravir concentration was increased to 400 mg/mL.

The suspension was prepared in a 250mL batch using a Netzsch miniCer and 0.3mm YTZ grinding beads. A range of excipient concentrations were explored.

The formulation vehicle was prepared by dissolving Polysorbate 20 (Croda), Polyethylene Glycol 3350 (Clariant), and mannitol (Roquette Freres) in water for injection (WFI) and filtering the solution through a 0.2µm filter. The formulation vehicle was then added to Cabotegravir (free acid) to prepare a 400 mg/ml coarse suspension. The coarse suspension, while stirred, was circulated through a wet bead mill set at 29.7Hz (Netzsch MiniCer) containing 0.30mm YTZ grinding beads (Nikkato Corp) at 73 - 145 ml/min until the desired median particle diameter of less than 0.25µm was reached as measured by laser diffraction. The wet bead mill was cooled to maintain a temperature between 1 and 25°C. The suspension was then filled into Type I glass vials, flushed with nitrogen, stoppered (FM457 stopper) and sealed. The filled suspension was terminally sterilized by gamma irradiation at a minimum dose of 25kGy.

30 mg/mL PS20, 30 mg/mL PEG3350 and 19 mg/mL mannitol with 400 mg/mL cabotegravir was prepared. The gamma irradiated suspension in vials was set down in the upright position and stored at 40°C/75%RH. After 1 month and 3 months of storage, suspensions were tested for stability. Table 16 shows particle size after formulation and after 1 month of storage at 40°C/75%RH for the 400 mg/mL cabotegravir formulation with 30 mg/mL PS20, 30 mg/mL PEG3350, 19 mg/mL mannitol. At 1 month, the particle size had increased compared to the initial time point (Table 13 and Figure 3). At 3 months, the suspension had become an unrecoverable gel which could not be removed from the vial with a syringe.

**Table 16**

| **Time** | **x10** | **x50** | **x90** |
|---|---|---|---|
| Initial | 0.07 | 0.17 | 0.61 |
| 1 month | 0.14 | 0.41 | 3.2 |

Further suspensions comprising cabotegravir, PS20 and PEG3350 were prepared with the concentrations shown in Table 17. Experiments 1, 2 and 5 were prepared using the method described above for 20 mg/mL PS20 and PEG3350. In experiment 3, the suspension was first milled with PS20 (no PEG3350), and then PEG3350 was added, here 400 mg/mL cabotegravir was milled with 30 mg/mL PS20. After milling, a concentrated solution (400 mg/mL) of PEG3350 was added to dilute the 400 mg/mL suspension to 360 mg/mL cabotegravir, 27 mg/mL PS20, 27 mg/mL mannitol. Compositions were all prepared on a 250mL batch scale using the Netzsch miniCer with 0.3mm YTZ grinding beads.

**Table 17**

| Experiment Number | PS20 (mg/mL) | PEG3350 (mg/mL) |
|---|---|---|
| 1 | 40.0 | 40.0 |
| 2 | 30.0 | 40.0 |
| 3 | 27* | 40.0 |
| 4 | 20 | 20.0 |
| 5 | 20 | 0 |

| | | |
|---|---|---|
| *400 mg/mL was diluted down by 10%, resulting in 360 mg/mL cabotegravir and 27 mg/mL PS20 | | |

In Experiments 1, 2, 4 and 5 during wet bead milling on the miniCer, the suspension thickened into a paste and was not recoverable. In Experiment 3, within 5 minutes of adding the 400 mg/mL PEG3350 solution, the suspension thickened and could no longer be stirred. The combination of PS20 and PEG3350 with 400 mg/mL cabotegravir did not produce a physically stable suspension.

### Example 9: 500 mg/mL Cabotegravir with Poloxamer 338 or 407

Injectable suspensions comprising about 500 mg/mL cabotegravir were prepared with Poloxamer 407 or Poloxamer 338; Suspension 4 and Suspension 5 are reference compositions. Mannitol was also added as a tonicity agent. The formulation vehicle was prepared by dissolving: 8.8g Poloxamer 338 and 7.0g mannitol in 159.3 g WFI (Suspension 4); 9.7g P338 (BASF) and 11.1g mannitol (Roquette Freres) in 159.3g WFI (Suspension 5); and 9.7g P407 (BASF) and 11.1g mannitol (Roquette Freres) in 159.3g WFI (Suspension 6) and filtering the solution through a 0.2µm filter. The formulation vehicle was added to 125g Cabotegravir (free acid) to prepare a 500 mg/ml coarse suspension (Suspension 4) or 138.8g Cabotegravir (free acid) to prepare a 555 mg/mL coarse suspension (Suspensions 5 & 6). The coarse suspension, while stirred, was circulated through a wet bead mill set at 29.7Hz (Netzsch MiniCer) containing 0.30mm YTZ grinding beads (Nikkato Corp) at 73 - 145 ml/min until the desired mean particle diameter of around 0.2µm was reached. The wet bead mill was cooled to maintain a temperature between 1 to 25°C. In the case of Suspensions 5 & 6, the milled suspension was diluted with concentrated PEG3350 solutions to produce final suspension compositions of 500 mg/mL Cabotegravir, 35 mg/mL P338 or P407, 40 mg/mL mannitol, and 0-40 mg/mL PEG3350. The suspension was filled into Type I glass vials, flushed with nitrogen, stoppered (FM457 stopper) and sealed. The resulting suspension was terminally sterilized by gamma irradiation at a minimum dose of 25kGy.

Table 18 shows the suspensions which were made. Each suspension was stored at 40°C/75%RH in an upright position. Initial particle size and particle size after 1 month was measured as an indicator of suspension stability. Table 19 shows the particle size in each suspension after 1 month. There was no change between initial particle size and particle size after 1 month. The lack of change in particle size is an indicator of stability of the suspensions.

The viscosity of each of the suspensions made in Table 18 was measured initially after they were made. The particle size of the suspensions was also measured initially after they were made as well as after 1 month of storage at 50°C. Results are shown in Table 19. Across all suspensions, there was minimal change in particle size after storage under accelerated conditions. The viscosity of Suspension 4 is within the range of viscosities observed in the Cabotegravir 400 mg/mL compositional DoE and is modestly higher than the viscosity of Suspension 3a (discussed above), which has a similar P338 composition and similar size target x50 of 0.2µm. The shear viscosity of the suspensions was measured using a Malvern Kinexus Pro rheometer equipped with a roughened cone and plate. Data was gathered at 20 °C with data collection at 10 points per decade across a shear rate range from 0.1 s-1 to 10 s-1.

**Table 18**

| | | | | |
|---|---|---|---|---|
| Suspension 4 | 497 mg/mL cabotegravir (measured by HPLC) | 35 mg/mL P338 | 40 mg/mL mannitol | 0 mg/mL PEG |
| Suspension 5 | 500 mg/mL cabotegravir (theoretical) | 35 mg/mL P338 | 40 mg/mL mannitol | 0-40 mg/mL PEG3350 |
| Suspension 6 | 500 mg/mL cabotegravir (theoretical) | 35 mg/mL P407 | 40 mg/mL mannitol | 0-40 mg/mL PEG3350 |

**Table 19**

| Suspension | Visc @ 0.1 s⁻¹ (cP) | Visc @ 10 s⁻¹ (cP) | **Initial** | | | **1 month** @ **50°C/ambient RH** | | |
|---|---|---|---|---|---|---|---|---|
| | | | X10 (um) | X50 (um) | X90 (um) | X10 (um) | X50 (um) | X90 (um) |
| Suspension 4 | 197.016 | 61.419 | 0.07 | 0.22 | 0.72 | 0.09 | 0.24 | 0.69 |
| Suspension 5 | NT | NT | 0.07 | 0.17 | 0.42 | - | | |
| Suspension 6 | NT | NT | 0.09 | 0.21 | 0.51 | - | | |

### Example 10: Wettability study to define limits of Cabotegravir suspension strength

Wettability of Cabotegravir in 38 mg/mL P338, 35 mg/mL PEG3350, and 20 mg/mL mannitol was assessed to estimate the approximate concentration limit for Cabotegravir suspension in this vehicle. This was performed by adding increasing amounts of Cab to 5g of vehicle until a gross change in coarse suspension properties was observed. The results are summarized in Table 20 and show that the upper boundary of Cabotegravir solid suspension concentration in this vehicle is 600 mg/mL or less.

**Table 20**

| mg/mL Cabotegravir | Cababotegravir (g) | Vehicle* (g) | Gross viscosity |
|---|---|---|---|
| 396 | 2.63 | 5 | Similar to water |
| 500 | 3.57 | 5 | Similar to water |
| 565† | 4.33 | 5 | Similar to water |
| 580† | 4.50 | 5 | Boundary of thickening |
| 600† | 4.81 | 5 | Thicker, but flowable |
| 635† | 5.28 | 5 | Slightly paste-like |

| | | | |
|---|---|---|---|
| *38 mg/mL P338, 35 mg/mL PEG3350, 20 mg/mL mannitol †These values were calculated using densities approximated from lower concentrations | | | |

## Claims

1. A pharmaceutical composition comprising cabotegravir or a pharmaceutically acceptable salt thereof, polyethylene glycol 3350 and poloxamer 338,
wherein the pharmaceutical composition comprises a cabotegravir concentration of 350 to 600 mg/mL, a poloxamer concentration of 4 to 50 mg/mL and a polyethylene glycol concentration of 5 to 50 mg/mL and wherein the pharmaceutical composition is suitable for use as an injectable composition.

2. The pharmaceutical composition according to Claim 1, wherein the cabotegravir is present in the form of particles with a median particle diameter of about 0.1 to 10 µm.

3. The pharmaceutical composition according to Claim 2, wherein the cabotegravir is present in the form of particles with a median particle diameter of above 0.15 to 0.25 µm.

4. The pharmaceutical composition according to any preceding claim, wherein the pharmaceutical composition further comprises mannitol.

5. The pharmaceutical composition according to any preceding claim, wherein the pharmaceutical composition comprises a cabotegravir concentration of 350 to 500 mg/mL.

6. The pharmaceutical composition according to Claim 5, wherein the pharmaceutical composition comprises a cabotegravir concentration of 380 to 420 mg/mL.

7. The pharmaceutical composition according to Claim 6, wherein the pharmaceutical composition comprises a cabotegravir concentration of about 400 mg/mL.

8. The pharmaceutical composition according to any preceding claim, wherein the composition is a parenteral pharmaceutical composition.

9. A pharmaceutical composition defined in any of Claims 1 to 8 for use in the treatment or prevention of HIV.

10. The pharmaceutical composition for use according to Claim 9, wherein the pharmaceutical composition is administered to a human intramuscularly or subcutaneously.

11. The pharmaceutical composition for use according to either Claim 9 or Claim 10, wherein about 1 mL to about 3 mL of the pharmaceutical composition is administered to a human.

12. The pharmaceutical composition for use according to any of Claims 9 to 11, wherein the pharmaceutical composition is administered once every month, once every 2 months or once every 3 months.

13. The pharmaceutical composition for use according to any of Claims 9 to 12, wherein 1 to 1.5 mL of the pharmaceutical composition is administered subcutaneously once every month.

14. The pharmaceutical composition for use according to any of Claims 9 to 13, wherein 2.5 to 3mL of the pharmaceutical composition is administered intramuscularly once every 3 months.

15. A method of making a pharmaceutical composition according to any of Claims 1 to 8, wherein the method comprises contacting cabotegravir or a pharmaceutically acceptable salt thereof with polyethylene glycol and poloxamer.

16. A kit comprising a pharmaceutical composition according to any of Claims 1 to 8 which comprises cabotegravir or a pharmaceutically acceptable salt thereof, polyethylene glycol 3350 and poloxamer 338.

## Patentansprüche

1. Pharmazeutische Zusammensetzung, umfassend Cabotegravir oder ein pharmazeutisch akzeptables Salz davon, Polyethylenglykol 3350 und Poloxamer 338,
wobei die pharmazeutische Zusammensetzung umfasst eine Cabotegravir-Konzentration von 350 bis 600 mg/ml, eine Poloxamer-Konzentration von 4 bis 50 mg/ml und eine Polyethylenglykol-Konzentration von 5 bis 50 mg/ml, und wobei die pharmazeutische Zusammensetzung zur Verwendung als injizierbare Zusammensetzung geeignet ist.

2. Pharmazeutische Zusammensetzung gemäß Anspruch 1, wobei das Cabotegravir in Form von Partikeln mit einem mittleren Partikeldurchmesser von etwa 0,1 bis 10 µm vorliegt.

3. Pharmazeutische Zusammensetzung gemäß Anspruch 2, wobei das Cabotegravir in Form von Partikeln mit einem mittleren Partikeldurchmesser von etwa 0,15 bis 0,25 µm vorliegt.

4. Pharmazeutische Zusammensetzung gemäß irgendeinem der vorhergehenden Ansprüchen, wobei die pharmazeutische Zusammensetzung ferner Mannitol umfasst.

5. Pharmazeutische Zusammensetzung gemäß irgendeinem der vorhergehenden Ansprüche, wobei die pharmazeutische Zusammensetzung eine Cabotegravir-Konzentration von 350 bis 500 mg/ml umfasst.

6. Pharmazeutische Zusammensetzung gemäß Anspruch 5, wobei die pharmazeutische Zusammensetzung eine Cabotegravir-Konzentration von 380 bis 420 mg/ml umfasst.

7. Pharmazeutische Zusammensetzung gemäß Anspruch 6, wobei die pharmazeutische Zusammensetzung eine Cabotegravir-Konzentration von etwa 400 mg/ml umfasst.

8. Pharmazeutische Zusammensetzung gemäß irgendeinem der vorhergehenden Ansprüche, wobei die Zusammensetzung eine parenterale pharmazeutische Zusammensetzung ist.

9. Pharmazeutische Zusammensetzung gemäß mindestens einem der Ansprüche 1 bis 8 zur Verwendung bei der Behandlung oder Prävention von HIV.

10. Pharmazeutische Zusammensetzung zur Verwendung gemäß Anspruch 9, wobei die pharmazeutische Zusammensetzung einem Menschen intramuskulär oder subkutan verabreicht wird.

11. Pharmazeutische Zusammensetzung zur Verwendung gemäß einem der Ansprüche 9 oder 10, wobei etwa 1 ml bis etwa 3 ml der pharmazeutischen Zusammensetzung einem Menschen verabreicht werden.

12. Pharmazeutische Zusammensetzung zur Verwendung gemäß mindestens einem der Ansprüche 9 bis 11, wobei die pharmazeutische Zusammensetzung einmal pro Monat, einmal alle 2 Monate oder einmal alle 3 Monate verabreicht wird.

13. Pharmazeutische Zusammensetzung zur Verwendung gemäß mindestens einem der Ansprüche 9 bis 12, wobei 1 bis 1,5 ml der pharmazeutischen Zusammensetzung einmal im Monat subkutan verabreicht werden.

14. Pharmazeutische Zusammensetzung zur Verwendung gemäß mindestens einem der Ansprüche 9 bis 13, wobei 2,5 bis 3 ml der pharmazeutischen Zusammensetzung einmal alle 3 Monate intramuskulär verabreicht werden.

15. Verfahren zur Herstellung einer pharmazeutischen Zusammensetzung gemäß mindestens einem der Ansprüche 1 bis 8, wobei das Verfahren das Inkontaktbringen von Cabotegravir oder ein pharmazeutisch akzeptables Salz davon mit Polyethylenglykol und Poloxamer umfasst.

16. Kit, umfassend eine pharmazeutische Zusammensetzung gemäß mindestens einem der Ansprüche 1 bis 8, umfassend Cabotegravir oder ein pharmazeutisch akzeptables Salz davon, Polyethylenglykol 3350 und Poloxamer 338.

## Revendications

1. Composition pharmaceutique comprenant du cabotégravir ou un sel pharmaceutiquement acceptable de celui-ci, du polyéthylèneglycol 3350 et du poloxamère 338,
où la composition pharmaceutique comprend une concentration de cabotégravir de 350 à 600 mg/mL, une concentration de poloxamère de 4 à 50 mg/mL et une concentration de polyéthylèneglycol de 5 à 50 mg/mL et où la composition pharmaceutique est appropriée pour être utilisée comme composition injectable.

2. Composition pharmaceutique selon la revendication 1, où le cabotégravir est présent sous forme de particules avec un diamètre de particule médian d'environ 0,1 à 10 µm.

3. Composition pharmaceutique selon la revendication 2, où le cabotégravir est présent sous forme de particules avec un diamètre de particule médian de plus de 0,15 à 0,25 µm.

4. Composition pharmaceutique selon l'une quelconque des revendications précédentes, où la composition pharmaceutique comprend en outre du mannitol.

5. Composition pharmaceutique selon l'une quelconque des revendications précédentes, où la composition pharmaceutique comprend une concentration de cabotégravir de 350 à 500 mg/mL.

6. Composition pharmaceutique selon la revendication 5, où la composition pharmaceutique comprend une concentration de cabotégravir de 380 à 420 mg/mL.

7. Composition pharmaceutique selon la revendication 6, où la composition pharmaceutique comprend une concentration de cabotégravir d'environ 400 mg/mL.

8. Composition pharmaceutique selon l'une quelconque des revendications précédentes, où la composition est une composition pharmaceutique parentérale.

9. Composition pharmaceutique définie dans l'une quelconque des revendications 1 à 8 destinée à être utilisée dans le traitement ou la prévention du **VIH.**

10. Composition pharmaceutique destinée à être utilisée selon la revendication 9, où la composition pharmaceutique est administrée à un être humain par voie intramusculaire ou sous-cutanée.

11. Composition pharmaceutique destinée à être utilisée selon la revendication 9 ou la revendication 10, où environ 1 mL à environ 3 mL de la composition pharmaceutique sont administrés à un être humain.

12. Composition pharmaceutique destinée à être utilisée selon l'une quelconque des revendications 9 à 11, où la composition pharmaceutique est administrée une fois par mois, une fois tous les 2 mois ou une fois tous les 3 mois.

13. Composition pharmaceutique destinée à être utilisée selon l'une quelconque des revendications 9 à 12, où 1 à 1,5 mL de la composition pharmaceutique est administré par voie sous-cutanée une fois par mois.

14. Composition pharmaceutique destinée à être utilisée selon l'une quelconque des revendications 9 à 13, où 2,5 à 3 mL de la composition pharmaceutique sont administrés par voie intramusculaire une fois tous les 3 mois.

15. Procédé de production d'une composition pharmaceutique selon l'une quelconque des revendications 1 à 8, où le procédé comprend la mise en contact du cabotégravir ou d'un sel pharmaceutiquement acceptable de celui-ci avec le polyéthylèneglycol et le poloxamère.

16. Kit comprenant une composition pharmaceutique selon l'une quelconque des revendications 1 à 8 qui comprend du cabotégravir ou un sel pharmaceutiquement acceptable de celui-ci, du polyéthylèneglycol 3350 et du poloxamère 338.
